# EUROPEAN PATENT APPLICATION

(11) **EP 3 108 883 A1**
(43) Date of publication of application: **28.12.2016**
(21) Application number: 15382332.3
(22) Date of filing: 22.06.2015
(51) Int. Cl.: A61K 31/18, A61K 31/381, A61K 31/4045, A61K 31/444, A61K 31/445, A61P 37/06

(54) **THERAPEUTIC USES OF NON-PEPTIDE INHIBITORS OF THE CALCINEURIN - NFAT SIGNALLING PATHWAY**

(71) Applicant: Fundació Institut de Recerca Biomèdica de Bellvitge, 08907 Hospitalet del Llobregat-Barcelona (ES); Universitat Autònoma de Barcelona, 08193 Barcelona (ES)
(72) Inventor: PÉREZ RIBA, Mercè, 08907 Hospitalet del Llobregat-Barcelona (ES); ARANGUREN IBÁÑEZ, Álvaro, 08907 Hospitalet del Llobregat-Barcelona (ES); MATSOUKAS, Minos-Timotheos, 08193 Bellaterra- Barcelona (ES); PARDO CARRASCO, Leonardo, 08193 Bellaterra- Barcelona (ES)
(74) Representative: ABG Patentes, S.L.

(57) **Abstract**

The present invention relates to sulphonamide compounds for their use in medicine and in the treatment of diseases in a subject such as inflammatory disease, an autoimmune disorder, a cardiovascular disease, a neurodegenerative disease, a disease occurring with uncontrolled cell proliferation and/or differentiation, an angiogenesis-related disease, an allergy, anaphylaxis and alopecia.

## Description

### TECHNICAL FIELD

The present invention relates to substituted sulphone compounds for their use in the treatment of diseases in a subject, wherein said diseases are diseases occurring with uncontrolled cell proliferation, immune disorders, cardiovascular disorders, neurodegenerative diseases, or other conditions in which an exacerbated activation of the calcineurin-NFAT pathway has been associated to its pathogenesis.

### BACKGROUND ART

Calcineurin (CN or PPP3, formerly PP2B) is a serine/threonine protein phosphatase regulated by Ca2+ and calmodulin. CN is a heterodimer formed by a calmodulin-binding catalytic subunit (calcineurin A, CNA) and a Ca2+-binding regulatory subunit (calcineurin B, CNB). CN regulates development, memory, cardiac function, and immune response by dephosphorylating a variety of protein substrates, including the family of cytosolic Nuclear Factor of Activated T-cells (NFATc) transcription factors. Dephosphorylated NFATc proteins translocate into the nucleus where, in cooperation with other transcription factors, induce the expression of many cytokine and chemokine genes, leading to T cell activation. The CN docking site of NFATc is mainly formed by the PxIxIT motif, the main anchoring site, and the LxVP motif, to properly position the substrate phosphoresidue towards the catalytic active site.

The immunosuppressant drugs cyclosporin A (CsA), a cyclic undecapeptide derived from the fungus *Beauveria nivea,* and tacrolimus (FK506), a 23-membered macrolide synthesized by the bacteria *Streptomyces tsukubaensis,* are the cornerstone of immunosuppressive therapy. These drugs, in complex with endogenous immunophilins, sterically hinder the phosphatase activity of CN towards NFATc and all other substrates, by blocking the LxVP binding site of CN. As a consequence, despite their benefits, severe side effects, such as neurotoxicity, renal dysfunction, hypertension and diabetes, have been related to long-term administration.

Therefore, it is necessary to develop alternative strategies for disrupting Ca²⁺-calcineurin- NFAT signalling pathway that overcome the problems associated to the methods based on the use of currently available immunosupressants to be more specific for the calcineurin/NFAT pathway.

### BRIEF SUMMARY OF THE INVENTION

The invention relates to a compound of formula (I) 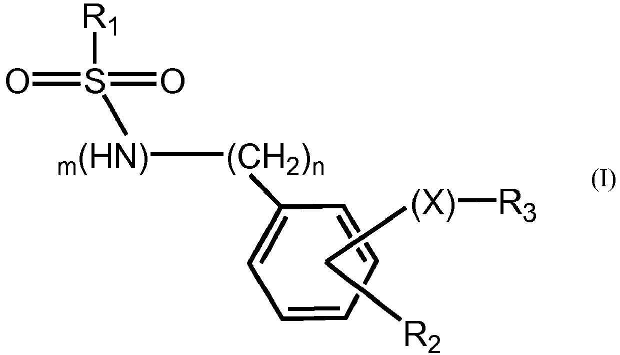 wherein:
R₁ is
   - hydrogen, halogen, hydroxy, trihalomethyl, trihalomethoxy, carbamoyl, phenyl or cyano, (C₁-C₈) alkyl, (C₁-C₈) alkylamino, (C₁-C₈) dialkylamino, (C₂-C₈) alkenyl, (C₂-C₈) alkenylamino, (C₂-C₈) dialkenylamino, (C₂-C₈) alkinyl, (C₂-C₈) alkinylamino, (C₂-C₈) dialkinylamino, alkoxy, aralkyl, alkanoyl,
   - a phenyl group or a phenylamino group, wherein the phenyl group is optionally substituted with 1, 2, 3, 4 or 5 substituents Y, which can be the same or different, and wherein Y is chosen from alkyl, alkoxy, halogen, hydroxy, trihalomethyl, trihalomethoxy, carbamoyl, phenyl and cyano,
   - a benzyl group or a bencylamine group, wherein the phenyl ring of the benzyl group is optionally substituted with 1, 2, 3, 4 or 5 substituents Y as defined above which can be the same or different,
   - a 5-membered carbocyclic or heterocyclic group optionally substituted with 1, 2, 3 or 4 substituents Y as defined above which can be the same or different,
   - a 6-membered carbocyclic or heterocyclic group optionally substituted with 1, 2, 3, 4 or 5 substituents Y as defined above which can be the same or different,
   - a group having the structure 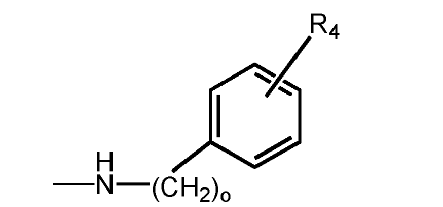 wherein o is an integer from 0 to 10 and R₄ is alkyl, alkoxy or halogen; or
   - a group having the structure 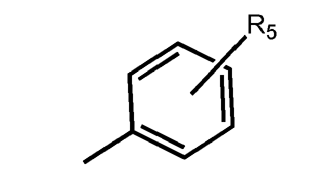 wherein R₅ is an alkyl group,
m is 0 or 1,
n is 0, 1 or 2,
X is -NH-CO- or -CO-NH-,
R₂ is hydrogen, halogen, alkyl, alkoxy, hydroxy, trihalomethyl, trihalomethoxy, carbamoyl, phenyl or cyano,
R₃ is
   - alkyl,
   - alkoxy or
   - a group having the structure -A-B
wherein
A is -(CH(R₆))-NH-, -CO-, -CH₂-CO-NH-, -CH(R₆)-NH-CO- or a direct bond, wherein R₆ is hydrogen or methyl and
B is an aromatic 5-membered carbocyclic or heterocyclic group optionally substituted with 1, 2, 3 or 4 substituents Y as defined above which can be the same or different or an aromatic 6-membered carbocyclic or heterocyclic group optionally substituted with 1, 2, 3, 4 or 5 substituents Y as defined above which can be the same or different, or wherein R₂ and R₃ form a heterocyclic group which is condensed to the phenyl group in formula (1) to form a bicyclic structure and wherein the heteroatom in the heterocycle is the nitrogen atom in the X group and wherein the carbonyl group of the X group is attached to a group having the structure -A-B, wherein A and B are as defined above, or a compound having the structure 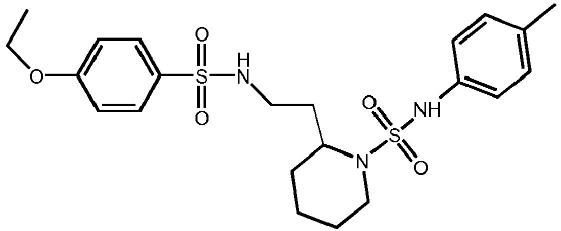 or a pharmaceutically acceptable active salt, hydrate, ester, solvate, prodrug, metabolite, stereoiosomer or mixtures thereof for use in the treatment of a disease in a subject, wherein said disease is selected from the group consisting of an inflammatory disease, an autoimmune disorder, a cardiovascular disease, a neurodegenerative disease, a disease occurring with uncontrolled cell proliferation and/or differentiation, an angiogenesis-related disease, an allergic disease, and alopecia.

### BRIEF DESCRIPTION OF THE FIGURES

**Figure 1****. Selected compounds 1-32 for experimental validation.**
**Figure 2****. Inhibiton of NFATc activity without blocking general phosphatase activity.**
   (A) Compounds 1-32 do not affect CN phosphatase activity using pNPP as substrate. Results are shown as percentage of CNA phosphatase activity in the presence of each compound relative to the same activity in the absence of any compound. (-) 50 µM ZnCl2. (B) Inhibition of the NFATc activity by compounds 1-32 *in vivo.* Luciferase reporter gene assays in HEK 293T cells after 24 h of treatment with compounds 1-32. Data are given as mean ± SEM of 3 independent experiments with triplicates. 100% of NFATc activation is achieved upon stimulation with ionomycin (Io), PMA and CaCl₂. (-) represents not stimulated cells; (+) cells stimulated with Io/PMA/Ca; (CsA) cells treated with CsA 30 min prior Io/PMA/CaCl₂ stimulation.

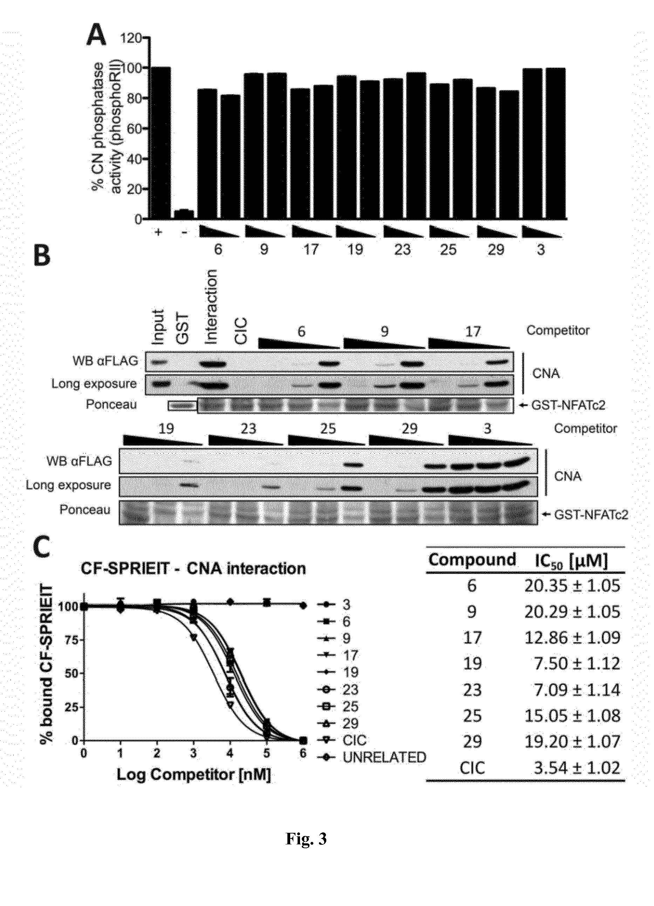

**Figure 3****. Compounds 6, 9, 17, 19, 23, 25 and 29 specifically bind to the PxIxIT binding region of Calcineurin and disrupt the CNA-NFATc2 interaction without blocking general Calcineurin phosphatase activity.**
   (**A**) CN phosphatase activity assay towards RII phosphopeptide in presence of 5 and 50 µM of each compound. (+) CN phosphatase activity in the absence of compound. (-) CN activity inhibition by EGTA was used as a negative control. (B) Competitive pull-down assays using GST-NFATc2 as bait and FLAG-CNA as CN source. The inhibitory compounds were added to the protein extracts at 500, 100 and 20 µM for 30 min. Western blot anti-FLAG was used to detect recombinant human CNA protein. GST was used as negative control. Ponceau staining shows equal GST fusion protein loading. (C) Competition binding studies with the NFATc2-derived SPRIEIT peptide by fluorescence polarization. RCAN3 CIC-derived peptide and an unrelated peptide were included as positive and negative controls, respectively. Data are represented as mean percentage of CF-SPRIEIT bound to CNA (± SEM) of three independent experiments performed in triplicates. Signal in the absence of competing peptide was considered as 100% of CF-SPRIEIT bound to CNA. Apparent IC50 values for all the competitions are displayed on the right side of the graph. The CIC peptide is used as positive control of NFATc2 competition and the unrelated compound as negative control. All data were obtained from triplicates of at least three independent experiments.

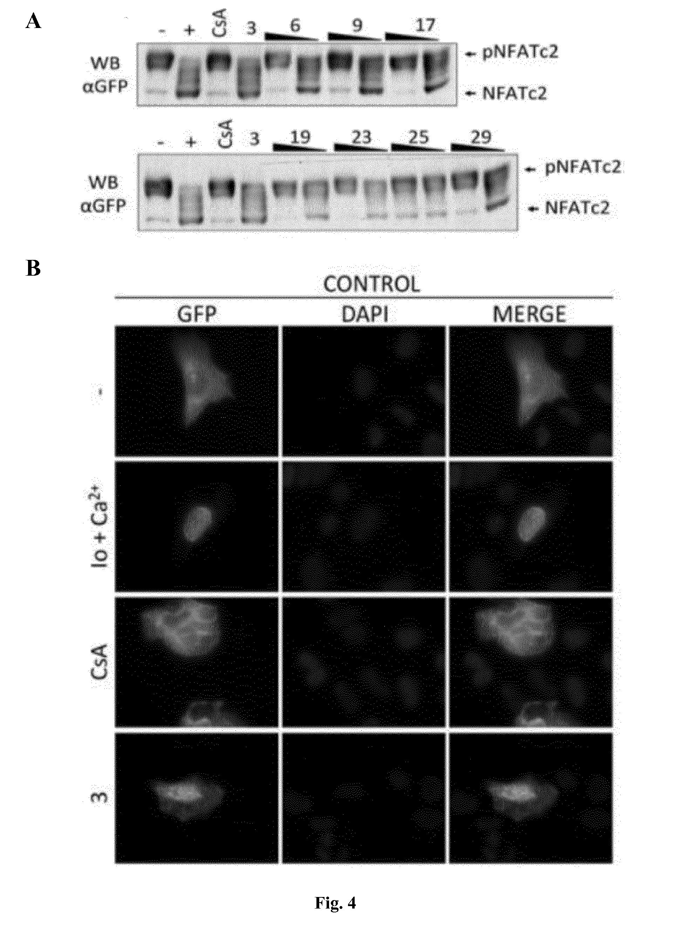

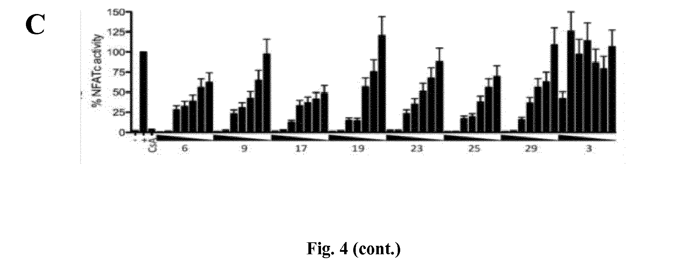

**Figure 4****. Compounds 6, 9,17,19, 23, 25 and 29 inhibit NFATc dephosphorylation, translocation into the nucleus and NFATc-dependent promoter activation.**
   HEK 293T (A) or U2OS (B) cells were transfected with GFP-HA-NFATc2 vector and treated with increasing concentrations of compounds 6, 9, 17, 19, 23, 25 and 29 for 2 h prior to stimulation with Ionomycin and CaCl2 for 30 min. Compound 3 was used as a negative control of NFATc activation. (**A**) Inhibition of GFP-NFATc2 dephosphorylation. (-) not stimulated cells. (Io+Ca2+) stimulated cells. (CsA) cells treated with CsA 30 min prior Io/PMA stimulation; (3) 100 µM of compound 3. (B) Inhibition of GFP-NFATc2 translocation from cytosol into the nucleus. Cell nuclei were stained with DAPI (blue). (C) Dose-response assays of inhibition of NFATc activity in HEK 293T cells transfected with 3xNFAT-luc plasmid. Data are represented as mean percentage, relative to 100% obtained upon stimulation with ionomycin, PMA and CaCl2, of NFATc activity (± SEM) of three independent experiments. (-) not stimulated cells; (+) stimulated with Io/PMA/Ca; (CsA) cells treated with CsA 30 min prior stimulation with Io/PMA/Ca/CsA.

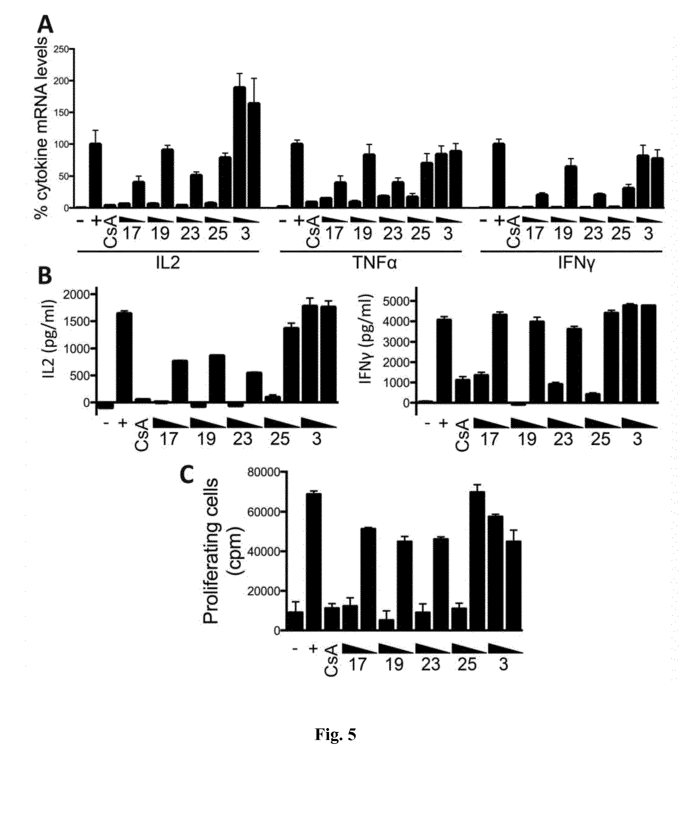

**Figure 5****. Compounds 17, 19, 23 and 25 strongly inhibit NFATc-dependent cytokine production and proliferation of activated human CD4+ T cells.**
   **(A)** Inhibition of NFATc-dependent gene expression by selected compounds was assessed by real time PCR for IL2, IFNγ and TNFα cytokines in compound pretreated isolated human CD4+ peripheral blood lymphocytes. **(B)** Inhibition of NFATc-dependent cytokine production in CD4+ human peripheral blood lymphocytes. NFATc-dependent IL-2 and IFNγ production was assessed in media from CD4+ hPBLs treated with selected compounds by enzyme-linked immunosorbent assay (ELISA) after CD3/CD28 stimulation. **(C)** Dose dependent inhibition of CD4+ hPBLs cell proliferation measured at 72 h.

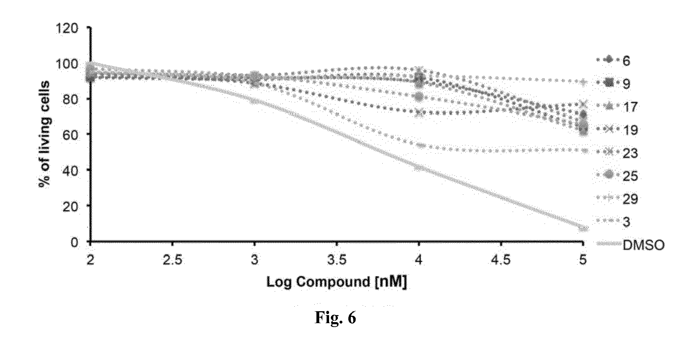

**Figure 6****. The selected compounds are not cytotoxic in eukaryotic cells.**
   MTT cytotoxicity assays in human HEK 293T cells. Cells (1.2 x 10₄ cells/well) were seeded in 96-well plates and 16 h later treated with serial dilutions from 100 µM to 0.1 µM of selected compounds per triplicate. After 24 h of treatment, 5 mM DTT was added to each well and incubated for 3 h prior to cell lysis with ispropanol-HCL (96:1). Absorbance at 560 nm was measured on a Victor™ X5 2030 Multilabel Reader (PerkinElmer Life Sciences). Background data was subtracted to all measurements and maximum cell viability was referred to values obtained with 0.1% DMSO treatment. All data were obtained from three independent experiments. DMSO was used as a control of cell cytotoxic compound. Data are represented as percentage of living cells in the presence of a compound referred to the absence of any compound. All data were obtained from three independent experiments.

### DETAILED DESCRIPTION OF THE INVENTION

The authors of the present invention have found substituted benzosulfone compounds which are capable of inhibiting NFATc activity without affecting general calcineuring phosphatase activity, of disrupting the interaction between calcineurin and the transcription factor NFAT, of preventing translocation into the nucleus of NFATc and NFATc-dependent promoter activation and of inhibiting NFATc-dependent cytokine production and proliferation of activated human CD4+ T cells (see examples).

Thus, in a first aspect, the invention relates to a compound of formula (I) 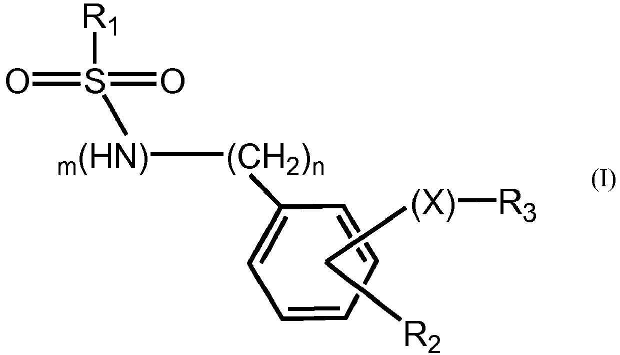 wherein:
R₁ is
   - hydrogen, halogen, hydroxy, trihalomethyl, trihalomethoxy, carbamoyl, phenyl or cyano,
   - (C₁-C₈) alkyl,
   - (C₁-C₈) alkylamino,
   - (C₁-C₈) dialkylamino,
   - (C₂-C₈) alkenyl,
   - (C₂-C₈) alkenylamino,
   - (C₂-C₈) dialkenylamino,
   - (C₂-C₈) alkinyl,
   - (C₂-C₈) alkinylamino,
   - (C₂-C₈) dialkinylamino,
   - alkoxy,
   - aralkyl,
   - alkanoyl,
   - a phenyl group or a phenylamino group, wherein the phenyl group is optionally substituted with 1, 2, 3, 4 or 5 substituents Y, which can be the same or different, and wherein Y is chosen from alkyl, alkoxy, halogen, hydroxy, trihalomethyl, trihalomethoxy, carbamoyl, phenyl and cyano,
   - a benzyl group or a benzylamine group, wherein the phenyl ring of the benzyl group is optionally substituted with 1, 2, 3, 4 or 5 substituents Y as defined above which can be the same or different,
   - a 5-membered carbocyclic or heterocyclic group optionally substituted with 1, 2, 3 or 4 substituents Y as defined above which can be the same or different,
   - a 6-membered carbocyclic or heterocyclic group optionally substituted with 1, 2, 3, 4 or 5 substituents Y as defined above which can be the same or different,
   - a group having the structure 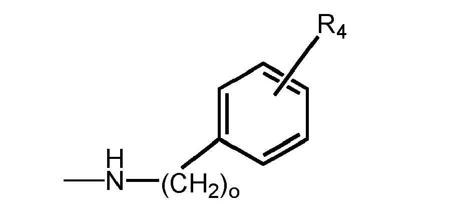 wherein o is an integer from 0 to 10, and R₄ is alkyl, alkoxy or halogen; or
   - a group having the structure 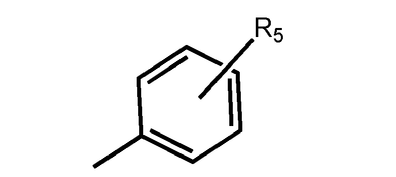 wherein R₅ is an alkyl group,
m is 0 or 1,
n is 0, 1 or 2,
X is -NH-CO- or -CO-NH-,
R₂ is hydrogen, halogen, alkyl, alkoxy, hydroxy, trihalomethyl, trihalomethoxy, carbamoyl, phenyl or cyano,
R₃ is
   - alkyl,
   - alkoxy or
   - a group having the structure -A-B
      wherein
      A is -(CH(R₆))-NH-, -CO-, -CH₂-CO-NH-, -CH(R₆)-NH-CO- or a direct bond, wherein R₆ is hydrogen or methyl, and
      B is an aromatic 5-membered carbocyclic or heterocyclic group optionally substituted with 1, 2, 3 or 4 substituents Y as defined above which can be the same or different or an aromatic 6-membered carbocyclic or heterocyclic group optionally substituted with 1, 2, 3, 4 or 5 substituents Y as defined above which can be the same or different, or wherein R₂ and R₃ form a heterocyclic group which is condensed to the phenyl group in formula (1) to form a bicyclic structure and wherein the heteroatom in the heterocycle is the nitrogen atom in the X group, said heteroatom being substituted with a group having the structure -A-B, wherein A and B are as defined above, or a compound having the structure 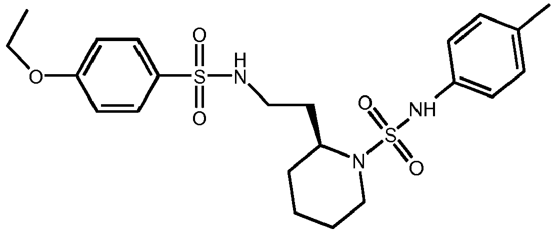 or a pharmaceutically acceptable salt, hydrate, ester, solvate, prodrug, metabolite, stereoiosomer or mixtures thereof for use in the treatment of a disease in a subject, wherein said disease is selected from the group consisting of an inflammatory disease, an autoimmune disorder, a cardiovascular disease, a neurodegenerative disease, a disease occurring with uncontrolled cell proliferation and/or differentiation, an angiogenesis-related disease, an allergy, anaphylaxis and alopecia.

As used herein, the term "C₁-C₈ alkyl group" refers to a saturated straight chain or branched non-cyclic hydrocarbon having from 1 to 8 carbon atoms. Representative saturated straight chain alkyls include methyl, ethyl, n-propyl, n-butyl, n-pentyl, n-hexyl; while saturated branched alkyls include isopropyl, sec-butyl, isobutyl, tert-butyl, isopentyl, 2-methylbutyl, 3-methylbutyl, 2-methylpentyl, 3-methylpentyl, 4-methylpentyl, 2,3-dimethylbutyl, n-heptyl, n-octyl and the like. Alkyl groups included in compounds of this invention may be optionally substituted with one or more substituents.

As used herein, the term "C₁-C₈ alkylamino group" refers to a saturated straight chain or branched non-cyclic hydrocarbon having from 1 to 8 carbon atoms as defined above and which are connected to the sulfone group by a primary or secondary amino group, thereby forming a sulphonamide group. The alkyl groups in the alkylamino groups included in compounds of this invention may be optionally substituted with one or more substituents. The amino group in the alkylamino groups included in compounds described herein may be further substituted with an additional alkyl group, in which case the group is known as dialkylamino group.

As used herein, the term "C₂-C₈ alkenyl group", means a saturated straight chain or branched non-cyclic hydrocarbon having from 2 to 8 carbon atoms and having at least one carbon-carbon double bond. Representative straight chain and branched (C2-C6) alkenyls include vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, 1-hexenyl, 2-hexenyl, 3-hexenyl, 1-heptenyl, 2-heptenyl, 3-heptenyl, 4-heptenyl, 5-heptenlyl, 6-heptenyl, 1-octyl, 2-octyl, 3-octyl, 4-octyl, 5-octy, 6-octyl, 7-octyl and the like. Alkenyl groups may be optionally substituted with one or more substituents.

As used herein, the term "C₂-C₈ alkenylamino group" refers to a saturated straight chain or branched non-cyclic hydrocarbon having from 2 to 8 carbon atoms and having at least one carbon-carbon double bond as defined above and which are connected to the sulfone group by a primary or secondary amino group, thereby forming a sulphonamide group. Alkenylamino groups included in compounds of this invention may be optionally substituted with one or more substituents. The amino group in the alkenylamino groups included in compounds described herein may be further substituted with an additional alkyl group, in which case the group is known as dialkenylamino group.

The term "C₂-C₈ alkynyl" as used herein means an unsaturated straight or branched chain aliphatic group with one or more carbon-carbon triple bonds, having from 2-8 carbon atoms, and preferably 2-6 carbon atoms. Preferred alkynyl groups include, without limitation, ethynyl, propynyl, butynyl, pentynyl, hexynyl, heptinyl and octinyl.

As used herein, the term "C₂-C₈ alkinylamino group" refers to a saturated straight chain or branched non-cyclic hydrocarbon having from 2 to 8 carbon atoms and with one or more carbon-carbon triple bonds as defined above and which are connected to the sulfone group by a primary or secondary amino group, thereby forming a sulphonamide group. Alkinylamino groups included in compounds of this invention may be optionally substituted with one or more substituents. The amino group in the alkinylamino groups included in compounds described herein may be further substituted with an additional alkinyl group, in which case the group is known as dialkinylamino group

As used herein, the term "alkoxy group" refers to an alkyl group which is attached to another moiety via an oxygen linker. Non-limiting examples of alkoxy groups include: -OCH₃ (methoxy), -OCH₂CH₃ (ethoxy), -OCH₂CH₂CH₃, -OCH(CH₃)₂ (isopropoxy), -OCH(CH2)₂, -O-cyclopentyl, and -O-cyclohexyl.

As used herein, the term "aralkyl" or "alkylaryl" refers to an aryl group bonded directly to an alkyl group, such as benzyl or phenethyl. The substituent may be further substituted by halo, hydroxy, alkyl, alkoxy, aryl, substituted aryl, substituted alkyl or aralkyl.

The term "alkanoyl," as used herein, represents a hydrogen or an alkyl group (e.g., a haloalkyl group) that is attached to the parent molecular group through a carbonyl group and is exemplified by formyl (i.e., a carboxyaldehyde group), acetyl, propionyl, butyryl, isobutyryl, and the like. Exemplary unsubstituted alkanoyl groups include from 1 to 7 carbons. In some embodiments, the alkyl group is further substituted with 1, 2, 3, or 4 substituents as described herein.

As used herein, "phenyl or phenylamine group optionally substituted with 1, 2, 3, 4 or 5 substituents Y", refers to a phenyl group which contains at least one substitution and wherein said substitution is at any desired place in the phenyl ring except at the carbon which attached to the nitrogen group in the benzosulfonamide moiety. As it will be understood, if the phenyl group contains one substituent, this may be found at positions ortho- meta- or para- with respect to the atom which is connected to the sulfone group. If the phenyl group contains two substituents, these may be located at the two ortho-positions, at the two meta-positions or at the para-position and at the meta- or ortho-positions. Including within this definition are included phenyl, 2-chlorophenyl, 3-chlorophenyl, 4-chlorophenyl, 2-fluorophenyl, 3-fluorophenyl, A-fluorophenyl, 2-bromophenyl, 3-bromophenyl, 4-bromophenyl, 2-trifluoromethylphenyl, 3- trifluoromethylphenyl, 4-trifluoromethylphenyl, 2-methoxyphenyl, 3-methoxyphenyl, A- methoxyphenyl, 2-aminophenyl, 3-aminophenyl, 4-aminophenyl, 2-methylaminophenyl, 3-methylaminophenyl, 4-methylaminophenyl, 2-dimethylaminophenyl, 3-dimethylaminophenyl, 4-dimethylaminophenyl, 2-methylphenyl, 3-methylphenyl, 4-methylphenyl, 2-nitrophenyl, 3- nitrophenyl, 4-nitrophenyl, 2,4-dichlorophenyl, 2,3-dichlorophenyl, 3,5-dimethylphenyl, 2-trifluoromethoxyphenyl, 3-trifluoromethoxyphenyl, 4-trifluoromethoxyphenyl, 2-phenoxyphenyl, 3-phenoxyphenyl, 4-phenoxyphenyl, 2-benzyloxyphenyl, 3-benzyloxyphenyl, 4-benzyloxyphenyl, 4-methoxycarbonylphenyl, 2-cyano-4,5-dimethoxyphenyl, 2-fluoro-3-trifluorophenyl, 3-fluoro- 5-trifluorophenyl, 2-fluoro-6-trifluoromethylphenyl, 2,4-dimethoxyphenyl, 4-hydroxy-3- methoxyphenyl, 3,5-dichloro-2-hydroxyphenyl, 3-bromo-4,5-dimethoxyphenyl, 4-benzyloxy- 3-methylphenyl, 3-benzyloxy-4-methylphenyl and the like.

As used herein, the term "halogen" or "halo" means -F, -Cl, -Br or -I.

As used herein, the term hydroxy refers to an -OH group.

As used herein the term "trihalomethyl" refers to a -CX₃ group wherein X is an halogen as defined above. In a preferred embodiment, the trihalomethyl group is a trifluoromethyl group.

As used herein the term "trihalomethyloxy" refers to a -CX₃-O- group wherein X is an halogen as defined above. In a preferred embodiment, the trihalomethyloxy group is a trifluoromethyloxy group.

As used herein, the term "carbamoyl" refers to a -O-CO-NRₓR_{y} group or -NRₓ-CO-O-R_{z}, wherein Rₓ, R_{y}, R_{z} can be aliphatic, aryl, araliphatic, heterocycloaliphatic, heteroaryl, or heteroaraliphatic.

As used herein, the term cyano is the -CN group.

As used herein, "benzyl group or benzylamine optionally substituted with 1, 2, 3, 4 or 5 substituents Y", refers to a benzyl or to a benzylamine group which contains at least one substitution and wherein said substitution is at any desired place in the phenyl ring forming part of the benzyl or benzylamine group except at the carbon which is connected to the sulfone group. As it will be understood, if the phenyl group contains one substituent, this may be found at positions ortho- meta- or para- with respect to the atom which is connected to the sulfone group. If the phenyl group contains two substituents, these may be located at the two ortho-positions, at the two meta-positions or at the para-position and at the meta- or ortho-positions.

As used herein, the term "5-membered carbocyclic or heterocyclic group optionally substituted with 1, 2 or 3 substituents Y" refers to any stable monocyclic, bicyclic or tricyclic carbon ring of five members in each ring, wherein at least one of the rings is saturated or unsaturared (aromatic) and unsubstituted or substituted with from one to three substituents Y as defined above. The 5 membered group is connected to the parent molecular moiety through any carbon atom or any heteroatom contained within the cyclic group. Representative examples of 5-membered heterocyclic groups include, but are not limited to, furyl, imidazolyl, isoxazolyl, isothiazolyl, oxadiazolyl, oxazolyl, pyrazolyl, pyrrolyl, tetrazolyl, thiadiazolyl, thiazolyl, thienyl, triazolyl, and triazinyl.

As used herein, the term "6-membered carbocyclic or heterocyclic group optionally substituted with 1, 2, 3 or 4 substituents Y" refers to any stable monocyclic, bicyclic or tricyclic carbon ring of six members in each ring, wherein at least one of the rings is saturated or unsaturared (aromatic) and unsubstituted or substituted with from one to four substituents Y as defined above. The 6 membered group is connected to the parent molecular moiety through any carbon atom or any heteroatom contained within the cyclic group. Representative examples of 6-membered heterocyclic groups include, but are not limited to, pyridinyl, pyridazinyl, pyrimidinyl, pyrazinyl, piperidinyl, oxanyl, thianyl and thiopyranyl.

The 5- or 6-membered carbocyclic or heterocyclic group optionally substituted may be aromatic and be condensed to a second ring, resulting in a bicyclic heteroaryl. Said second ring may be consists of a monocyclic heteroaryl fused to a phenyl, or a monocyclic heteroaryl fused to a cycloalkyl, or a monocyclic heteroaryl fused to a cycloalkenyl, or a monocyclic heteroaryl fused to a monocyclic heteroaryl. The bicyclic heteroaryl is connected to the parent molecular moiety through any carbon atom or any nitrogen atom contained within the bicyclic heteroaryl. Representative examples of bicyclic heteroaryl include, but are not limited to, benzimidazolyl, benzofuranyl, benzothienyl, benzoxadiazolyl, cinnolinyl, dihydroquinolinyl, dihydroisoquinolinyl, furopyridinyl, indazolyl, indolyl, isoquinolinyl, naphthyridinyl, quinolinyl, tetrahydroquinolinyl, and thienopyridinyl.

In any of the embodiments above, whenever a range of the number of atoms in a structure is indicated (e.g., a C₁-C₈), it is specifically contemplated that any sub-range or individual number of carbon atoms falling within the indicated range also can be used. Thus, for instance, the recitation of a range of 1-8 carbon atoms (e.g., C₁-C₈) as used with respect to any chemical group (e.g., alkyl, aryl, etc.) referenced herein encompasses and specifically describes 1, 2, 3, 4, 5, 6, 7 and/or 8 carbon atoms, as appropriate, as well as any sub-range thereof (e.g., 1-2 carbon atoms, 1-3 carbon atoms, 1-4 carbon atoms, 1-5 carbon atoms, 1-6 carbon atoms, 1-7 carbon atoms, 1-8 carbon atoms, 2-3 carbon atoms, 2-4 carbon atoms, 2-5 carbon atoms, 2-6 carbon atoms, 2-7 carbon atoms, 2-8 carbon atoms, 3-4 carbon atoms, 3-5 carbon atoms, 3- 6 carbon atoms, 3-7 carbon atoms, 3-8 carbon atoms, 4-5 carbon atoms and 4-6 carbon atoms, 3-7 carbon atoms and 3-8 carbon atoms as appropriate.

In any of the embodiments above, the presence of a double bond is indicative that the molecule can be as *cis* or *trans* isomer.

The term "cis-trans isomers", which are sometimes referred to as geometrical isomers, occur when double bonds prevent rotation of atoms around a bond. In a cis-isomer (Z isomer according to the E-Z notation), both of the larger groups lie on the same side of the molecule. In the trans-isomer (E isomer, according to the E-Z notation), the larger groups are on opposite sides of the molecule.

In another embodiment the isomer resulting from the double bond connecting the thiazolidindione and the ring carrying the R₃ group is the Z isomer. In another embodiment, the isomer resulting from the double bond connecting the thiazolidinedione ring and the ring carrying the R₃ group is the E isomer. In another embodiment, the compounds of the invention are provided as mixture of the E and Z isomer.

In some embodiments, the compound of formula (1) for use according to the present invention has an R₁ group which is a N-piperidine group. In a preferred embodiment, the N-piperidine group is unsubstituted.

In a preferred embodiment, the compound of formula (1) for use according to the present invention has an R₁ group which is a N-piperidine group, preferably unsubstituted, an X group which is -NH-CO-, an R₂ group which is an halogen, preferably chlorine, m is 0, n is 0 and an R₃ group which has a -A-B structure wherein A is -(CH(R₆))-NH- and R₆ is as defined above, preferably, methyl group, and B is a 6-membered heterocyclic group optionally substituted with 1, 2, 3, 4 or 5 substituents Y as defined above which can be the same or different, preferably 1 substituent and, more preferably, a methoxy group and, even more preferably, the-A-B in the R₃ group has the structure

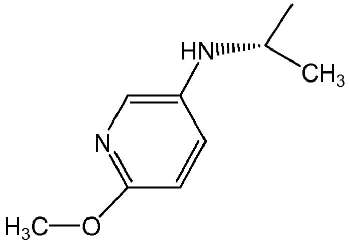

In some embodiments, the compound for use according to the invention has an R₁ group which is an alkylamino group. In a preferred embodiment, the alkylamino group is a C₁-C₈ alkylamino group. In a more preferred embodiment, the alkylamino group is an ethylamino group.

In a preferred embodiment, the alkylamino group is an ethylamino group, m and n are both 0, the X group is -CO-NH, the R₂ group is hydrogen and the R₃ group has the structure

-A-B

wherein the A element in the -A-B group is -CH₂-CO-NH- and the B element group contains 2 Y substituents, which are two identical halogen groups, preferably, fluorine atoms and more preferably, the -A-B group has the structure

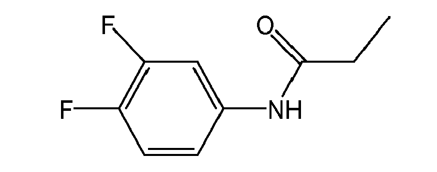

In some embodiments, the compound for use according to the invention has an R₁ group which is a dialkylamino group. In a preferred embodiment, the two alkyl groups in the dialkylamino group are identical. In some embodiments the two alkyl groups in the dialkylamino group are C₁-C₈ alkyl groups. In a more preferred embodiment, the dialkylamino group is a dimethylamino group.

In one embodiment, the alkylamino group is an dimethylamino group, m and n are both 0, the X group is -CO-NH-, R₂ is halogen, preferably chlorine and R₃ is a C₈ alkyl group, more preferably a branched C₈ alkyl group, and even more preferably a C₈ alkyl group having the structure

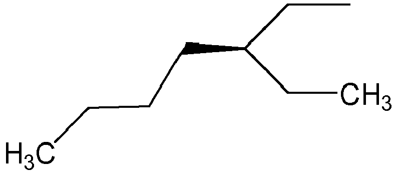

In some embodiments, the compound for use according to the invention has an R₁ group which has the following structure 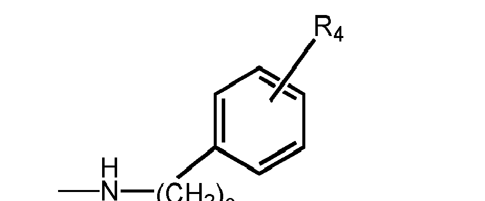 wherein o is an integer from 0 to 10 and wherein R₄ is an alkoxy group or an halogen. In some embodiments, o is 1 and R₄ is a methoxy group. In some embodiments, o is 0 and
R₄ is an halogen. In a still more preferred embodiment, the halogen is chlorine.

In some embodiments, the compound for use according to the invention has an R₁ group which has the following structure

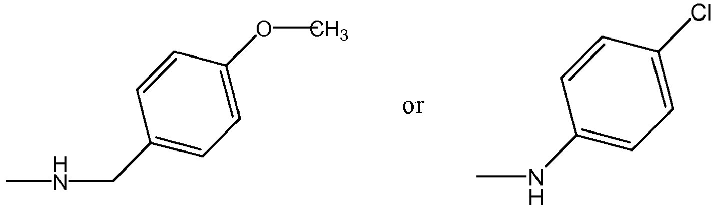

In some embodiments, the compound of formula (1) for use according to the present invention has an R₁ group which has the structure 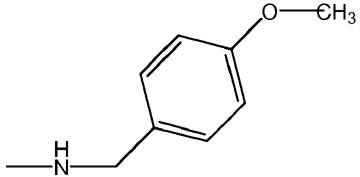 m and n are both 0, X is -CO-NH-, R₂ is halogen, preferably fluorine and R₃ has a-A-B structure wherein the A element in the -A-B group is a direct bond and the B group contains 2 Y substituents which are different, preferably halogen and alkyl, wherein the halogen is preferably fluorine and the alkyl is a methyl group and, even more preferably, wherein the -A-B group has the structure:

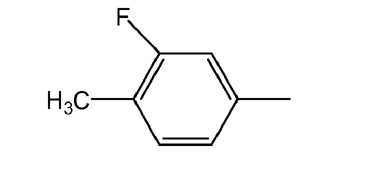

In some embodiments, the compound for use according to the invention has an R₁ group which has the structure 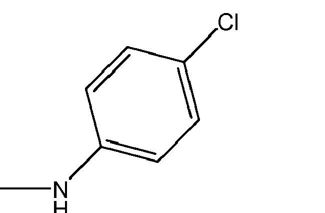 m and n are both 0, X is -NH-CO-, R₂ is halogen, preferably chlorine and R₃ has a-A-B structure wherein A is -CH(R₆)-NH-CO-, wherein R₆ is preferably methyl, and B is a 5-membered heteroaryl group optionally substituted with 1, 2, 3 or 4 substituents Y as defined above which can be the same or different, wherein the preferably the 5-membered heteroaryl group is a thiophene group containing a single Y substituent, preferably an alkyl group and even more preferably a methyl group. In a still more preferred embodiment, the -A-B group has the structure

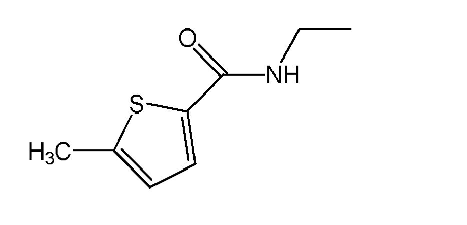

In some embodiments, the compound for use according to the invention has an R₁ group which has the following structure 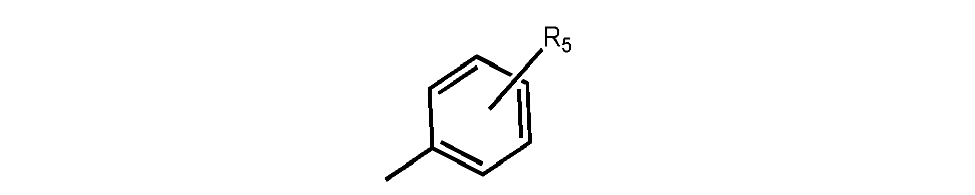 wherein R₅ is an alkyl group. In a preferred embodiment, the R₅ group is a C₁- C₈ alkyl group. In a more preferred embodiment, the R₅ group is a branched alkyl group. In a still more preferred embodiment, the R₅ group is an isopropyl group. In a preferred embodiment, the R₁ group has the structure

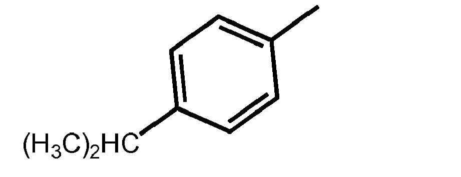

In a preferred embodiment, the R₁ group has the structure 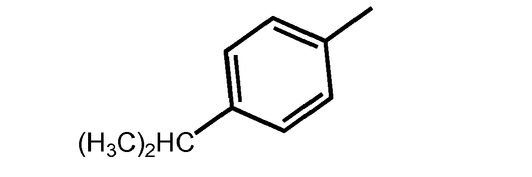 m and n are both 1, X is -NH-CO- and the R₂ and R₃ groups which form a heterocyclic group, preferably a saturated or unsaturated five membered ring, preferably a pyrrolidine which is condensed to the phenyl group in formula (I) to form a bicyclic structure, preferably a benzopyrrolidine and more preferably, a benzopyrrolidine wherein its phenyl part is not substituted, wherein the nitrogen atom in the X group is the heteroatom in the heterocycle, preferably the single heteroatom in the heterocycle and wherein the carbonyl group of the X group is attached to a group having the structure -A-B, wherein A and B are as defined above. In a preferred embodiment, the heterocycle is a saturated or unsaturated five membered ring. In some embodiments, the nitrogen atom in the X group is single heteroatom in the five membered ring and, even more preferably, the bicyclic structure has the following structure

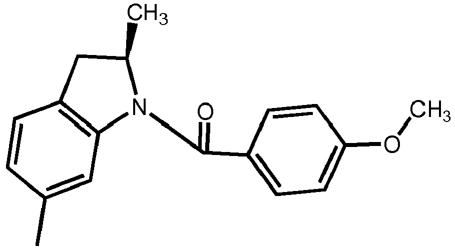

In some embodiments, the compound of formula (1) for use according to the present invention has a structure wherein m is 0, 1, 2, 3, or more. In some embodiments, the compound of formula (I) for use according to the present invention has a structure wherein n is 0, 1, 2, 3, or more. In some embodiments, the compound of formula (1) for use according to the present invention has a structure wherein both m and n 0 or 1 or wherein m is 1 and n is 2.

In some embodiments, the compound of formula (1) for use according to the present invention has an R₂ group which is hydrogen. In some embodiments, the compound of formula (1) for use according to the present invention has an R₂ group which is halogen. In a preferred embodiment, said halogen is chlorine or fluorine.

In some embodiments, the compound of formula (1) for use according to the inventions has an R₃ group having the structure -A-B wherein A is -(CH(R₆))-NH- and R₆ is as defined above, and B is a 6-membered heterocyclic group optionally substituted with 1, 2, 3, 4 or 5 substituents Y as defined above which can be the same or different.

In some embodiments, the -A-B in the R₃ group has the structure

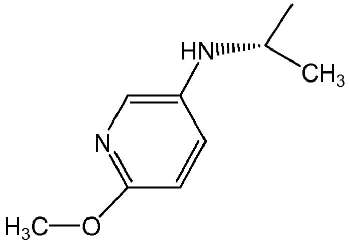

In some embodiments, the -A-B group consists of a -CH₂-CO-NH- or a direct bond as A element and a phenyl optionally substituted with 1, 2, 3, 4 or 5 substituents Y as defined above which can be the same or different as B element.

In some embodiments, the A element in the -A-B group is -CH₂-CO-NH- and the B element group contains 2 Y substituents, which are the same. In a preferred embodiment, the 2 Y substituents are halogen groups. In a more preferred embodiment, the 2 halogen atoms are fluorine atoms.

In some embodiments, the-A-B group has the structure

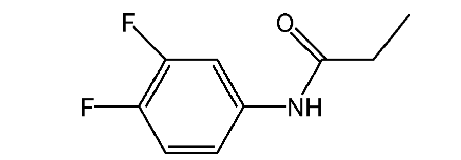

In some embodimemts, the A element in the -A-B group is a direct bond and the B group contains 2 Y substituents which are different. In a preferred embodiment, the 2 Y substituents are halogen and alkyl. In a more preferred embodiment, the halogen is a fluorine group. In a more preferred embodiment the alkyl is a methyl group. In a still more preferred embodiment, the -A-B group has the structure

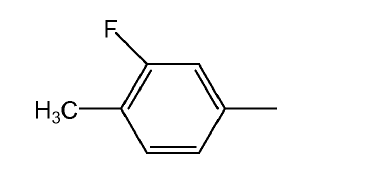

In some embodiments, the compound of formula (1) for use according to the present invention contains a R₃ group having the structure -A-B wherein A is-CH(R₆)-NH-CO- and B is a 5-membered heteroaryl group optionally substituted with 1, 2, 3 or 4 substituents Y as defined above which can be the same or different. In a preferred embodiment, the 5-membered heteroaryl group is a thiophene group containing a single Y substituent. In a more preferred embodiment, the single Y substituent is an alkyl group. In a more preferred embodiment, the alkyl group is a methyl group. In a still more preferred embodiment, the -A-B group has the structure

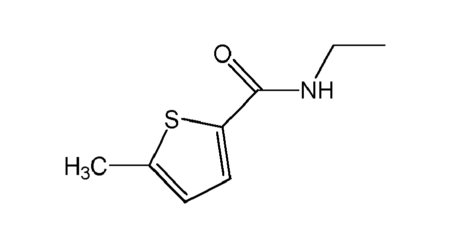

In some embodiments, the compound of formula (1) for use according to the invention has an R₃ group which is a C₈ alkyl group. In a preferred embodiment, the C₈ alkyl group is a branched C₈ alkyl group. In a more preferred embodiment, the C₈ alkyl group has the structure

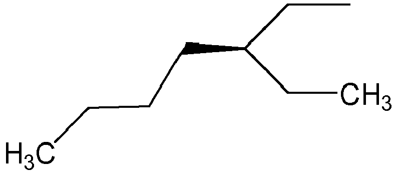

In some embodiments, the compound of formula (1) for use according to the invention contains R₂ and R₃ groups which form a heterocyclic group which is condensed to the phenyl group in formula (1) to form a bicyclic structure wherein the nitrogen atom in the X group is the heteroatom in the heterocycle and wherein the carbonyl group of the X group is attached to a group having the structure -A-B, wherein A and B are as defined above. In a preferred embodiment, the heterocycle is a saturated or unsaturated five membered ring. In some embodiments, the nitrogen atom in the X group is single heteroatom in the five membered ring. In some embodiments, the bicyclic structure is a benzopyrrolidine. In some embodiments, the phenyl part of the benzopyrrolidine is not substituted.

In a preferred embodiment, the bicyclic structure has the following structure

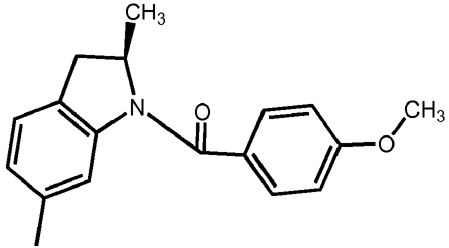

In some embodiments, the compound of formula (1) for use according to the present invention has a structure selected from the group consisting of

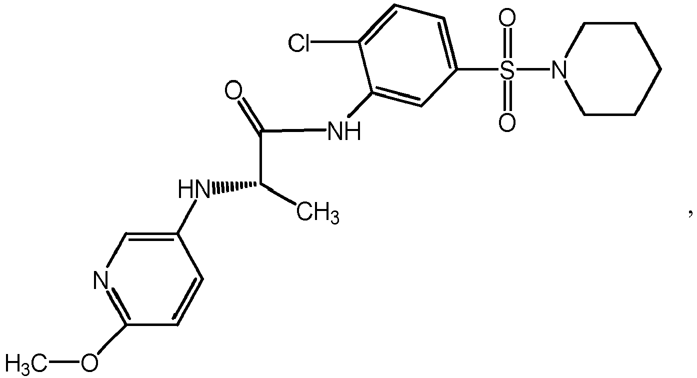

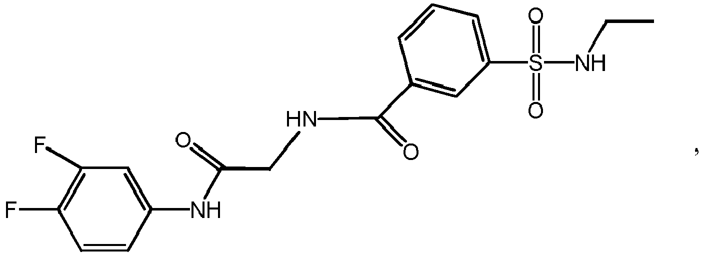

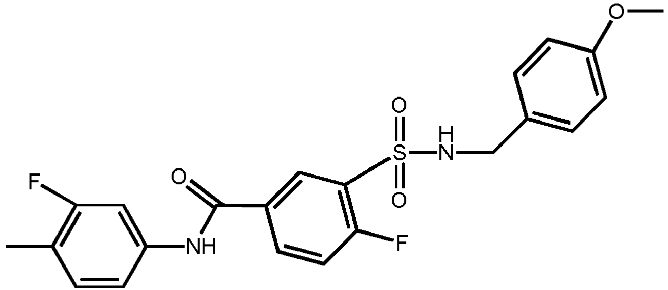

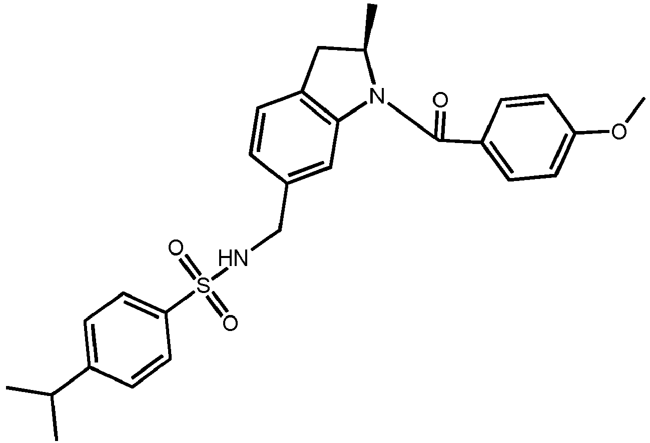

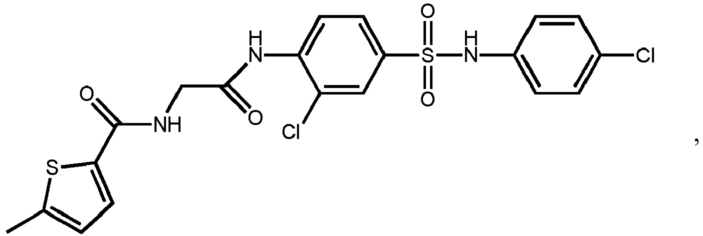

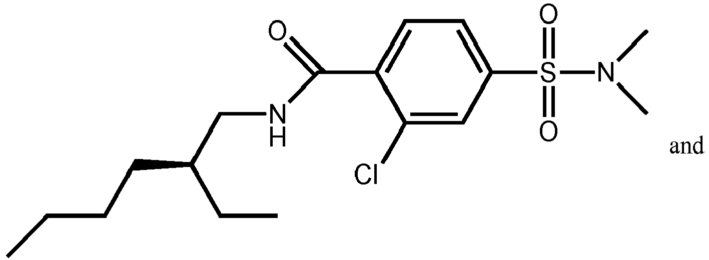

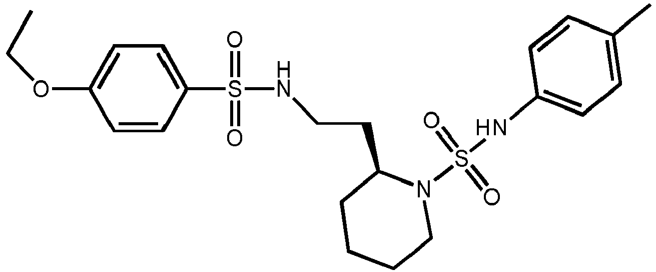

When a disclosed compound is named or depicted by structure, it is to be understood that solvates (e.g., hydrates) of the compound or its pharmaceutically acceptable salts are also included. "Solvates" refer to crystalline forms wherein solvent molecules are incorporated into the crystal lattice during crystallization. Solvate may include water or non-aqueous solvents such as ethanol, isopropanol, DMSO, acetic acid, ethanolamine, and EtOAc. Solvates, wherein water is the solvent molecule incorporated into the crystal lattice, are typically referred to as "hydrates". Hydrates include stoichiometric hydrates as well as compositions containing variable amounts of water.

When a disclosed compound is named or depicted by structure, it is to be understood that the compound, including solvates thereof, may exist in crystalline forms, non-crystalline forms or a mixture thereof. The compounds or solvates may also exhibit polymorphism (i.e. the capacity to occur in different crystalline forms). These different crystalline forms are typically known as "polymorphs." It is to be understood that when named or depicted by structure, the disclosed compounds and solvates (e.g., hydrates) also include all polymorphs thereof. Polymorphs have the same chemical composition but differ in packing, geometrical arrangement, and other descriptive properties of the crystalline solid state. Polymorphs, therefore, may have different physical properties such as shape, density, hardness, deformability, stability, and dissolution properties. Polymorphs typically exhibit different melting points, IR spectra, and X-ray powder diffraction patterns, which may be used for identification. One of ordinary skill in the art will appreciate that different polymorphs may be produced, for example, by changing or adjusting the conditions used in solidifying the compound. For example, changes in temperature, pressure, or solvent may result in different polymorphs. In addition, one polymorph may spontaneously convert to another polymorph under certain conditions.

The invention also provides "salts" of the compounds described in the present description. By way of illustration, said salts can be acid addition salts, base addition salts or metal salts, and can be synthesized from the parent compounds containing a basic or acid moiety by means of conventional chemical processes known by the persons skilled in the art. Such salts are generally prepared, for example, by reacting the free acid or base forms of said compounds with a stoichiometric amount of the suitable base or acid in water or in an organic solvent or in a mixture of the two. Non-aqueous media such as ether, ethyl acetate, ethanol, acetone, isopropanol or acetonitrile are generally preferred. Illustrative examples of said acid addition salts include inorganic acid addition salts such as, for example, hydrochloride, hydrobromide, hydroiodide, sulfate, nitrate, phosphate, etc., organic acid addition salts such as, for example, acetate, maleate, fumarate, citrate, oxalate, succinate, tartrate, malate, mandelate, methanesulfonate, p-toluenesulfonate, camphorsulfonate, etc. Illustrative examples of base addition salts include inorganic base salts such as, for example, ammonium salts and organic base salts such as, for example, ethylenediamine, ethanolamine, triethanolamine, glutamine, amino acid basic salts, etc. Illustrative examples of metal salts include, for example, sodium, potassium, calcium, magnesium, aluminum and lithium salts.

The invention also provides "esters" of the compounds described in the present description. It will be understood that the compounds defined herein may contain hydroxy group which may form an ester when the hydroxy group reacts with a carboxylic acid. Alternatively, the compounds defined herein may contain carboxylic acid groups which may form an ester when this group reacts with an alcohol. The term "ester " means an ester group which can be cleaved *in vivo* by a biological method such as hydrolysis and forms a compound as defined herein having a free carboxylic acid group or a free hydroxyl group or a salt thereof. In some embodiment, the ester is formed between a hydroxyl group in the compound of formula (I) and an carboxylic acid. Examples of such esters include C₁-C₄ alkoxy; benzyloxy; benzyloxy substituted on the phenyl ring with halogen, C₁-C₄ alkyl or C₁-C₄ alkoxy; C₁-C₅ alkanoyloxymethyl; or C₁-C₄ alkanoyloxy- methyl substituted on the oxomethyl with C₁-C₄ alkyl or C₄-C₇ cycloalkyl.

The invention also provides "prodrugs" of the compounds described in the present description. The term "prodrug", as used herein, is intended to represent covalently bonded carriers, which are capable of releasing the compound of formula (I) as active ingredient when the prodrug is administered to a mammalian subject. Release of the active ingredient occurs *in vivo.* Prodrugs can be prepared by techniques known to one skilled in the art. These techniques generally modify appropriate functional groups in a given compound. These modified functional groups however regenerate original functional groups by routine manipulation or *in vivo.* Prodrugs of compounds of the invention include compounds wherein a hydroxy, amino, carboxylic, or a similar group is modified. Examples of prodrugs include, but are not limited to esters (e.g., acetate, formate, and benzoate derivatives), carbamates (e.g., N,N-dimethylaminocarbonyl) of hydroxy or amino functional groups in compounds of Formula (I)), amides (e.g., trifluoroacetylamino, acetylamino, and the like), and the like

The invention also provides "metabolites" of the compounds described in the present description. A "metabolite" of a compound disclosed herein is a derivative of that compound that is formed when the compound is metabolized. The term "active metabolite" refers to a biologically active derivative of a compound that is formed when the compound is metabolized. The term "metabolized," as used herein, refers to the sum of the processes (including, but not limited to, hydrolysis reactions and reactions catalyzed by enzymes) by which a particular substance is changed by an organism. Thus, enzymes may produce specific structural alterations to a compound. For example, cytochrome P450 catalyzes a variety of oxidative and reductive reactions while uridine diphosphate glucuronyltransferases catalyze the transfer of an activated glucuronic-acid molecule to aromatic alcohols, aliphatic alcohols, carboxylic acids, amines and free sulphydryl groups. Metabolites of the compounds disclosed herein are optionally identified either by administration of compounds to a host and analysis of tissue samples from the host, or by incubation of compounds with hepatic cells *in vitro* and analysis of the resulting compounds.

The invention also provides "stereoisomers" of the compounds described in the present description. The term "stereoisomer," as used herein, refers to all possible different isomeric as well as conformational forms which a compound may possess (e.g., a compound of any formula described herein), in particular all possible stereochemically and conformationally isomeric forms, all diastereomers, enantiomers and/or conformers of the basic molecular structure. Some compounds of the present invention may exist in different tautomeric forms, all of the latter being included within the scope of the present invention.

It will be understood that the pharmaceutically acceptable salts, hydrates, esters, solvates, prodrugs, metabolites and stereoiosomers of the compound of formula (I) are active, i.e. they are capable of inhibiting the calcineurin-NFAT signalling pathway. Salts, hydrates, esters, solvates, prodrugs, metabolites and stereoiosomers capable of inhibiting the calcineurin-NFAT signalling pathway can be identified by any method known in the art for measuring calcineurin-induced activation of NFAT using a stimulant, e.g., calcium ionophore, a neurotransmitter, or a biologically active peptide, known to trigger activation of NFAT via the calcium/calcineurin pathway (for examples, see Table 1 in Rao et al, Annu. Rev. Immunol. 15:707-747 (1997)). Such methods include, without limitation, the method described in example 3 of the present invention based on the determination of the capability of the compounds to displace NFATc from the calcineurin interaction, the method described in example 4 of the application based on the determination of the capability of the compounds to inhibit dephosphorylation of NFATc in cells after induction of calcineurin activity by stimulation with ionomycin, PMA and CaCl₂, the method also shown in example 4 of the application based on the determination of the capability of the compounds to inhibit translocation of NFATc into the nucleus measured using, e.g. a fusion protein of NFATc and a fluorescent protein or the method described in example 5 of the application based on the determination of the capability of the compounds to inhibit proliferation of activated human primary CD4+ T-cells.

The compounds of formula (I) as well as the salts, hydrates, esters, solvates, prodrugs, metabolites and stereoiosomers thereof, while being capable of inhibiting the calcineurin-NFAT signalling pathway, do not affect the phosphatase activity of calcineurin. Calcineurin activity can be measured using standard assays based on the determination of the phosphatase activity on phosphopeptides which are known substrates of calcineurin such as the RII phosphopeptide.

It has been reported that the inhibition of NFAT signaling results in an immunosuppressive action, suppression of (heart) muscle hypertrophy (Wilkins BJ and Molkentin JD 2004 Biochem Biophys Res Commun 322:1178-1191), suppression of osteoclastic differentiation and decrease in cancer cell migration, invasion and proliferation (Muller MR and Rao A 2010 Nat Rev Immunol 10:645-656; Gachet S and Ghysdael 2009 J. Gen. Physiol. Biophys. Spec No F47-54). Thus, inhibitors of the NFAT transcription factor are useful in the treatment of a number of all diseases wherein it is desired to decrease the activity of NFAT. In view of the ability of the compounds of formula (I) to inhibit calcineurin-mediated NFAT signalling, the compounds as well as the salts, hydrates, esters, solvates, prodrugs, metabolites and stereoiosomers thereof are useful for the treating diseases which require inhibition of NFAT.

Thus, in another aspect, the invention relates to compounds as those previously described for their use in the treatment of diseases involving NFAT activation.

The term "disease involving NFAT activation", as used herein, refers to diseases which occur with increased NFAT activation, as determined using any of the methods mentioned above based on the determination of the transcriptional activity of NFAT, the phosphorylation status of NFAT or the cellular localization of NFAT. Diseases involving NFAT activation which can be treated with the compounds of formula (I) include, without limitation, an inflammatory disease, an autoimmune disorder, a cardiovascular disease, a neurodegenerative disease, a disease occurring with uncontrolled cell proliferation and/or differentiation, an angiogenesis-related disease, an allergy, anaphylaxis and alopecia.

Thus, in another aspect, the invention relates to a compound as previously described for use in the treatment of a disease in a subject, wherein said disease is selected from the group consisting of an inflammatory disease, an autoimmune disorder, a cardiovascular disease, a neurodegenerative disease, a diseases occurring with uncontrolled cell proliferation and alopecia.

Alternatively, the invention relates to a compound as previously described for the manufacture of a medicament for the treatment of a disease in a subject, wherein said disease is selected from the group comprising inflammatory diseases, autoimmune disorders, cardiovascular diseases, neurodegenerative diseases and diseases occurring with uncontrolled cell proliferation

Alternatively, the invention relates to a method for the treatment of a disease selected from the group consisting of inflammatory diseases, autoimmune disorders, cardiovascular diseases, neurodegenerative diseases and diseases occurring with uncontrolled cell proliferation in a subject in need thereof which comprises the administration to said subject of a compound as previously described.

As used herein, the term "treatment" refers to both therapeutic measures and prophylactic or preventative measures, wherein the object is to prevent or slow down (lessen) an undesired physiological change or disorder, such as the acute rejection after a renal transplant. Beneficial or desired clinical results include, but are not limited to, alleviation of symptoms, diminishment of extent of disease, stabilized (*i.e.,* not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

As used herein, the terms "prevent", "prevention" and "preventing" refer to the reduction in the risk of acquiring or developing a given disease or disorder, e.g., a proliferative disorder, or the reduction or inhibition of the recurrence or a disease or disorder, e.g., a proliferative disorder. In one embodiment, a compound of the invention is administered as a preventative measure to a patient, preferably a human, having a genetic predisposition to any of the disorders described herein.

By "subject" or "individual" or "animal" or "patient" or "mammal," is meant any subject, particularly a mammalian subject, for whom diagnosis, prognosis, or therapy is desired. Mammalian subjects include humans, domestic animals, farm animals, and zoo, sports, or pet animals such as dogs, cats, guinea pigs, rabbits, rats, mice, horses, cattle, cows, and so on. In a preferred embodiment of the invention, the subject is a mammal. In a more preferred embodiment of the invention, the subject is a human.

The term "inflammatory disease" refers to a condition in a subject characterized by inflammation, e.g., chronic inflammation. Illustrative, non-limiting examples of inflammatory disorders include, but are not limited to, Celiac Disease, rheumatoid arthritis (RA), Inflammatory Bowel Disease (IBD), asthma, encephalitis, chronic obstructive pulmonary disease (COPD), inflammatory osteolysis, allergic disorders, septic shock, pulmonary fibrosis (e.g. , idiopathic pulmonary fibrosis), inflammatory vacultides (e.g. , polyarteritis nodosa, Wegner's granulomatosis, Takayasu's arteritis, temporal arteritis, and lymphomatoid granulomatosus), post-traumatic vascular angioplasty (e.g. , restenosis after angioplasty), undifferentiated spondyloarthropathy, undifferentiated arthropathy, arthritis, inflammatory osteolysis, chronic hepatitis, and chronic inflammation resulting from chronic viral or bacteria infections.

The term "inflammatory disease" also includes immune mediated inflammatory disease, which shall be taken to mean any disease mediated by the immune system and characterized by chronic or acute inflammation, resulting from, associated with or triggered by, a dysregulation of the normal immune response e.g. Crohn's disease, type 1 diabetes mellitus, rheumatoid arthritis, inflammatory bowel disease, psoriasis, psoriatic arthritis, ankylosing spondylitis, systemic lupus erythematosus, Hashimoto's disease, graft-versus-host disease, Sjogren's syndrome, pernicious anemia, Addison disease, scleroderma, Goodpasture's syndrome, ulcerative colitis, autoimmune hemolytic anemia, sterility, myasthenia gravis, multiple sclerosis, Basedow's disease, thrombopenia purpura, Guillain-Barré syndrome, allergy, asthma, atopic disease, arteriosclerosis, myocarditis, cardiomyopathy, glomerular nephritis, hypoplastic anemia, and rejection after organ transplantation.

The term "autoimmune disorder" or "autoimmune disease" refers to a condition in a subject characterized by cellular, tissue and/or organ injury caused by an immunological reaction of the subject to its own cells, tissues and/or organs. Illustrative, non-limiting examples of autoimmune diseases which can be treated with the compounds of formula (I) include alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac sprue-dermatitis, chronic fatigue immune dysfunction syndrome (CF1DS), chronic inflammatory demyelinating polyneuropathy, Churg-Strauss syndrome, cicatrical pemphigoid, CREST syndrome, cold agglutinin disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, Meniere's disease, mixed connective tissue disease, multiple sclerosis, type 1 or immune-mediated diabetes mellitus, myasthenia gravis, pemphigus vulgaris, pernicious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, sarcoidosis, scleroderma, progressive systemic sclerosis, Sjogren's syndrome, Good pasture's syndrome, stiff-man syndrome, systemic lupus erythematosus, lupus erythematosus, takayasu arteritis, temporal arteristis/giant cell arteritis, ulcerative colitis, uveitis, vasculitides such as dermatitis herpetiformis vasculitis, vitiligo, Wegener's granulomatosis, Anti-Glomerular Basement Membrane Disease, Antiphospholipid Syndrome, Autoimmune Diseases of the Nervous System , Familial Mediterranean Fever, Lambert-Eaton Myasthenic Syndrome, Sympathetic Ophthalmia, polyendocrinopathies, psoriasis, etc.

The term "cardiovascular disease or disorder", as used herein, relates to diseases affecting the heart or blood vessels or both or associated with the cardiopulmonary and circulatory systems including but not limited to ischemia, angina, edematous conditions, artherosclerosis, Coronary Heart Disease, LDL oxidation, adhesion of monocytes to endothelial cells, foam-cell formation, fatty-streak development, platelet adherence, and aggregation, smooth muscle cell proliferation, reperfusion injury, high blood pressure, thrombotic disease, arrhythmia (atrial or ventricular or both); cardiac rhythm disturbances; myocardial ischemia; myocardial infarction; cardiac or vascular aneurysm; vasculitis, stroke; peripheral obstructive arteriopathy of a limb, an organ, or a tissue; reperfusion injury following ischemia of the brain, heart or other organ or tissue, endotoxic, surgical, or traumatic shock; hypertension, valvular heart disease, heart failure, abnormal blood pressure; shock; vasoconstriction (including that associated with migraines); vascular abnormality, inflammation, insufficiency limited to a single organ or tissue.

In a preferred embodiment of the invention, the cardiovascular disease is cardiac hypertrophy or atherosclerotic plaque instability.

The term "neurodegenerative disease", as it is used herein, is related to diseases which result from the degeneration or deterioration of nervous tissue, particularly of neurons, leading over time to a dysfunction or to a disability; the term degeneration includes loss of cell viability, loss of cell function and/or loss of the number of cells (neurons or others). Illustrative, non-limiting, examples of neurodegenerative diseases include Alzheimer's disease, Huntington's disease, Parkinson's disease, amyotrophic lateral sclerosis (ALS), multiple sclerosis, etc. In a particular embodiment, said neurodegenerative disease is a disease related to neuronal death caused by a substance which, for example, causes oxidative stress or endoplasmic reticulum stress or apoptosis or excitotoxicity or neuronal death in general.

The term "disease occurring with uncontrolled cell proliferation and/or differentiation" is related to diseases characterized by disregulation of the mechanisms controlling cell division. An ilustrative, non-limiting, example of a disease occurring with uncontrolled cell proliferation includes cancer.

The term "cancer" is referred to a disease characterized by uncontrolled cell division (or by an increase of survival or apoptosis resistance) and by the ability of said cells to invade other neighbouring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumours are classified as being either benign or malignant: benign tumours are tumours that cannot spread by invasion or metastasis, i.e., they only grow locally; whereas malignant tumours are tumours that are capable of spreading by invasion and metastasis. As used herein, the term cancer includes, but is not limited to, the following types of cancer: breast cancer; biliary tract cancer; bladder cancer; brain cancer including glioblastomas and medulloblastomas; cervical cancer; choriocarcinoma; colon cancer; endometrial cancer; esophageal cancer; gastric cancer; hematological neoplasms including acute lymphocytic and myelogenous leukemia; T-cell acute lymphoblastic leukemia/lymphoma; hairy cell leukemia; chronic myelogenous leukemia, multiple myeloma; AIDS-associated leukemias and adult T-cell leukemia/lymphoma; intraepithelial neoplasms including Bowen's disease and Paget's disease; liver cancer; lung cancer; lymphomas including Hodglun's disease and lymphocytic lymphomas; neuroblastomas; oral cancer including squamous cell carcinoma; ovarian cancer including those arising from epithelial cells, stromal cells, germ cells and mesenchymal cells; pancreatic cancer; prostate cancer; rectal cancer; sarcomas including leiomyosarcoma, rhabdomyosarcoma, liposarcoma, fibrosarcoma, and osteosarcoma; slun cancer including melanoma, Merkel cell carcinoma, Kaposi's sarcoma, basal cell carcinoma, and squamous cell cancer; testicular cancer including germinal tumors such as seminoma, non-seminoma (teratomas, choriocarcinomas), stromal tumors, and germ cell tumors; thyroid cancer including thyroid adenocarcinoma and medullar carcinoma; and renal cancer including adenocarcinoma and Wilms tumor. Other cancers will-be known to one of ordinary skill in the art.

In a preferred embodiment of the invention, the disease occurring with uncontrolled cell proliferation is cancer. In a more preferred embodiment of the invention, the cancer is selected from the group consisting of breast cancer, colon cancer, prostate cancer and immunological cancer.

The term "breast cancer" or "breast carcinoma" refers to any malignant proliferative mammary cell disorder, usually occurring in the ducts (the tubes that carry milk to the nipple) and lobules (milk producing glands).

The term "colon cancer" or "colon carcinoma" refers to any malignant proliferative colon cell disorder.

The term "prostate cancer" or "prostate carcinoma" refers to any malignant proliferative prostate cell disorder.

The term "immunological cancer" or "immunological carcinoma" refers to any malignant proliferative immune system cell disorder.

The term "angiogenesis-related disease" refers to diseases caused by the dysregulation of the processes mediating angiogenesis. In particular, abnormal angiogenesis associated disease refers to atherosclerosis, hypertension, tumor growth, cancer, inflammation, rheumatoid arthritis, wet-form macular degeneration, choroidal neovascularization, retinal neovascularization, abnormal angiogenesis in diabetic subjects, myocardial edema, pulmonary edema and diabetic retinopathy.

The term "allergic disease" as used herein refers to any symptoms, tissue damage, or loss of tissue function resulting from allergy and includes, without limitation, diseases such as atopic dermatitis, urticaria, contact dermatitis, allergic conjunctivitis, allergic rhinitis, allergic asthma, anaphylaxis, food allergy and hay fever.

In a preferred embodiment, the allergic reaction is anaphylaxis. The term "anaphylaxis", as used herein, refers to an acute, systemic allergic reaction that occurs after an individual has become sensitized to an antigen. It can be associated with the production of high levels of immunoglobulin E (IgE) antibodies and/or with the release of histamines, which can cause muscle contractions, constriction of the airways (bronchospasm), and vasodilation. Symptoms of anaphylactic and/or anaphylactoid reactions can include hives, generalized itching, nasal congestion, wheezing, difficulty breathing, cough, cyanosis, lightheadedness, dizziness, confusion, slurred speech, rapid pulse, palpitations, nausea and vomiting, abdominal pain or cramping, skin redness or inflammation, nasal flaring, intercostals retractions, etc. Possible complications of severe anaphylactic and/or anaphylactoid reactions can include airway blockage, cardiac arrest, respiratory arrest, and/or shock.

The term "alopecia" includes the involuntary complete or partial hair loss from the head or body of an individual and includes alopecia areata (AA), alopecia totalis (AT), alopecia universalis (AU), androgenetic alopecia (alopecia androgenetica, or male baldness) or post- chemotherapy alopecia (PCA) or chemotherapy-induced alopecia (CIA). Alopecia areata may include diffuse alopecia areata, alopecia areata monolocularis, alopecia areata multilocularis, and alopecia areata barbae.

It will be understood that the therapeutic uses according to the present invention require the administration of an effective amount of the compounds described herein. As used herein, the term "effective amount" refers to an amount of a compound of this invention which is sufficient to reduce or ameliorate the severity, duration, progression, or onset of a disease or disorder, e.g., a proliferative disorder, prevent the advancement of a disease or disorder, e.g., a proliferative disorder, cause the regression of a disease or disorder, e.g., a proliferative, prevent the recurrence, development, onset or progression of a symptom associated with a disease or disorder, e.g., a proliferative disorder, or enhance or improve the prophylactic or therapeutic effect(s) of another therapy. The precise amount of compound administered to a subject will depend on the mode of administration, the type and severity of the disease or condition and on the characteristics of the subject, such as general health, age, sex, body weight and tolerance to drugs. It will also depend on the degree, severity and type of cell proliferation, and the mode of administration. The skilled artisan will be able to determine appropriate dosages depending on these and other factors. When co-administered with other agents, e.g., when co-administered with an anti-cancer agent, an "effective amount" of the second agent will depend on the type of drug used. Suitable dosages are known for approved agents and can be adjusted by the skilled artisan according to the condition of the subject, the type of condition(s) being treated and the amount of a compound of the invention being used. In cases where no amount is expressly noted, an effective amount should be assumed.

Non-limiting examples of an effective amount of a compound of the invention are provided herein below. In a specific embodiment, the invention provides a method of preventing, treating, managing, or ameliorating a proliferative disorder or one or more symptoms thereof, said methods comprising administering to a subject in need thereof a dose of at least about 150 µg/kg, preferably at least about 250 µg/kg, at least about 500 µg/kg, at least about 1 mg/kg, at least about 5 mg/kg, at least about 10 mg/kg, at least about 25 mg/kg, at least about 50 mg/kg, at least about 75 mg/kg, at least about 100 mg/kg, at least about 125 mg/kg, at least about 150 mg/kg, or at least about 200 mg/kg or more of one or more compounds of the invention once every day, preferably, once every 2 days, once every 3 days, once every 4 days, once every 5 days, once every 6 days, once every 7 days, once every 8 days, once every 10 days, once every two weeks, once every three weeks, or once a month.

It will be understood that the uses according to the present invention contemplate the administration of the compounds forming part of a pharmaceutical composition comprising a therapeutically effective amount of the compound of the invention together with at least one pharmaceutically acceptable excipient.

The pharmaceutical composition of the invention can be administered by any suitable route of administration, for example, oral, by inhalation, parenteral (for example, subcutaneous, intraperitoneal, intravenous, intramuscular route, etc.), rectal route, etc.

Illustrative examples of dosage forms for administration by the oral route include tablets, capsules, granulate, solutions, suspensions, etc., and can contain the conventional excipients, such as binders, diluents, disintegrants, lubricants, wetting agents, etc., and can be prepared by conventional methods. The pharmaceutical compositions can also be adapted for their parenteral administration, in the form of, for example, sterile solutions, suspensions or lyophilized products, in the suitable dosage form; in this case, said pharmaceutical compositions will include the suitable excipients, such as buffers, surfactants, etc. In any case, the excipients will be chosen according to the selected pharmaceutical dosage form.

A review of the different dosage forms for the administration of drugs and their preparation can be found in the book "Tratado de Farmacia Galénica", by C. Faulí i Trillo, 10 Edition, 1993, Luzán 5, S.A. de Ediciones.

The composition of the invention is administered in a manner compatible with the dosage formulation and in a therapeutically effective amount. The quantity to be administered and timing depends on the subject to be treated, capacity of the subject's system to utilize the active ingredient, and degree of therapeutic effect desired. Precise amounts of active ingredient required to be administered depend on the judgment of the practitioner and are peculiar to each individual.

As used herein, the terms "pharmaceutically acceptable", "physiologically tolerable" and grammatical variations thereof, as they refer to compositions, carriers, diluents and reagents, are used interchangeably and represent that the materials are capable of administration to or upon a mammal without the production of undesirable physiological effects.

A "pharmaceutically acceptable excipient," "pharmaceutically acceptable diluent," or "pharmaceutically acceptable carrier", or "pharmaceutically acceptable vehicle," used interchangeably herein, refer to a non-toxic solid, semisolid or liquid filler, diluent, encapsulating material or formulation auxiliary of any conventional type. A pharmaceutically acceptable excipient is essentially non-toxic to recipients at the employed dosages and concentrations and is compatible with other ingredients of the formulation. For example, the carrier for a formulation containing polypeptides would not normally include oxidizing agents and other compounds that are known to be deleterious to polypeptides. Suitable carriers include, but are not limited to water, dextrose, glycerol, saline, ethanol, and combinations thereof. The carrier can contain additional agents such as wetting or emulsifying agents, pH buffering agents, or adjuvants that enhance the effectiveness of the formulation.

The term "pharmaceutically acceptable salt" refers to any pharmaceutically acceptable salt, (isomer, solvate, prodrug or any other compound) which, upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. The preparation of salts (isomers, solvates, prodrugs and derivatives) can be carried out by methods known in the art.

For instance, pharmaceutically acceptable salts of compounds provided herein are synthesized from the parent compound which contains a basic moiety by conventional chemical methods. Generally, such salts are, for example, prepared by reacting the free base forms of these compounds with a stoichiometric amount of the appropriate acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate.

The invention is described in detail below by means of the following examples which are to be construed as merely illustrative and not limitative of the scope of the invention.

### EXAMPLES

### Materials and Methods

### Structure-based pharmacophore model

Discovery Studio 3.5 software was used for the generation of the pharmacophore model using the crystal structure of the AKAP79-derived peptide and CNA (PDB id 3LL8) (14) as the starting structure. Water molecules in the crystal structure were removed for the analysis. Crucial interaction patterns between the peptide and CNA were determined using a minimum feature distance of 2 Å. The obtained pharmacophore was edited manually to contain a total of twelve pharmacophoric features. 864 different pharmacophores, containing at least seven of these features, were created because small, non-peptidic, molecules cannot fulfill these twelve features. These 864 pharmacophores contain at least three out of the five hydrophobic features, at least two out of the five hydrogen bonds involving backbone protein - peptide interactions, and the hydrogen bond acceptor between Thr11 of the peptide and Asn330 of CNA. Excluded features were manually added to represent the van der Waals surface ofCNA.

### A pharmacophore-based virtual screen

The about 12.4 million compounds of the Clean Drug-like subset of the ZINC database were filtered according to molecular weight (350-600Da), AlogP (1-6), hydrogen bond acceptors (≥3), hydrogen bond donors (≥1), and aromatic groups (≥1) using Discovery Studio 3.5 and Openbabel, finally leading to ∼5.5 million compounds. These compounds were fitted to the 864 different pharmacophore models using Discovery Studio 3.5. The fit value scoring function was used to rank the compounds from best to worst fitting.

### Quantum mechanical/molecular mechanical structure optimization

A combined QM/MM method, using Gromacs v4.5.5 and ORCA v2.9.1, was used to determine the interaction between 17, 19, 23 and 25 and CNA. The QM region is composed by -strand of CN formed by all atoms of Met329, Asn330, Ile331, Arg332, and Gln333, and the side chain atoms of Ile303, Tyr288, Met290, Phe299, and Pro300 of CN. The MM region is formed by all the other atoms of CN. The B3LYP/6-31G(d) method was used for the QM layer and the AMBER99SB force field was used for the MM layer.

### Plasmid constructs, proteins production and purification

The 3xNFAT-luc reporter plasmid, the pRL-null plasmid, which lacks a promoter driving Renilla expression, the GST-NFATc2 and the FLAG-hCNAα 2-389, HA-NFATc2-GFP and pGEX-6P-hCNAα (amino acids 2-347) were produced as previously described (Mulero et al., J.Biol.Chem., 2009, 284: 9394-9401; Mulero, et al., 2007, BBA Mol. Cell Res. 1773: 330-341 and Martinez-Hoyer et al., 2013, BBA Mol. Cell Res. 1833, 2311-2321). GST-hCNα (amino acids 2-347) and GST-NFATc2 fusion proteins were produced as reported (Martinez-Hoyer et al., 2013, supra., and Aubareda et al., 2006,Cell. Signal. 18: 1430-1438).

### In vitro CNA phosphatase assay

CN activity towards the pNPP substrate was measured as described (McAvoy, et al., in Current Protocols in Molecular Biology. (John Wiley & Sons, Inc., 2001), vol. 92, chap. 18, pp. 18). The CN phosphatase assay kit (ENZO Life Sciences, Farmingdale, NY) was used to assess the dephosphorylation of phosphoRII peptide by CN (Mulero et al., 2009, supra.).

### NFATc signaling assays

Human HEK 293T and U2OS cell lines were grown and maintained in DMEM with Glutamax media (Invitrogen) supplemented with 10% FBS and transfected with polyethyleneimide (PEI) (29). Assays were carried out 24 h post transfection. NFATc activity, NFATc2 subcellular localization and competitive GST-NFATc2 pull-down assays were performed as previously described ((Martinez-Hoyer et al., 2013, supra.,).

### Fluorescence polarization

Competition assays of the CNA-SPRIEIT (NFATc2 derived peptide: ASGLSPRIEITPSHEL; SEQ ID NO: 1) interaction, by fluorescence polarization, were performed using 10 µM of human CNAα (amino acids 2-347), 10 nM of carboxyfluorescein (CF)-tagged NFATc2-derived SPRIEIT peptide (SEQ ID NO: 1) (CF-SPRIEIT) and different concentrations of compounds 6, 9, 17, 19, 23, 25 and 29 as previously described (18). The RCAN3178-203 (CIC) peptide (KYELHAGTESTPSVVVHVCES; SEQ ID NO: 2) was used as positive control of NFATc competition for binding to CNA.

### MTT cytotoxicity essays

Human HEK 293T (1.2 x 104 cells/well) were seeded in 96-well plates. The next morning cells were treated with serial dilutions from 100 µM to 0.1 µM selected compounds per triplicate. After 24 h, 5 mM DTT was added, incubated for 3 h and lysed with ispropanol-HCL (96:1), and read absorbance at 560 nm on a Victor™ X5 2030 Multilabel Reader (PerkinElmer Life Sciences). Background data was subtracted to all measurements and maximum cell viability was referred to values obtained with 0.1% DMSO treatment.

### NFATc2 dephosphorylation assay

HEK 293T cells transfected with HA-NFATc2-GFP plasmid were treated for 2 h with 10 and 100 µM of compounds 6, 9, 17, 19, 23, 25 and 29 and stimulated with ionomycin and CaCl2 (see Supporting Materials and Methods). Cells were lysed in RIPA buffer, resolved on 7.5% SDS-PAGE, and analyzed by western blot using anti-EGFP antibody.

### Human T cell proliferation and cytokine production assays

The CD4+ T cells were purified using automacs CD4 microbead human kit II (Miltenyi) according to manufacturer's instructions. CD4+ T cells were seeded in 96-plate and treated with the indicated inhibitory compounds at specified concentrations for 2 h. Next, the lymphocytes were activated with anti-CD3/anti-CD28 beads (Dynabeads human T-activator CD3/CD28, Gibco) and after 5 h of incubation, mRNA was purified, retrotranscribed and analyzed by real-time PCR as previously described (32). In parallel, IL2 and IFNγ production was measured by ELISA (BD Pharmingen) in supernatants collected after 48 h of culture, following manufacturer's instructions. T cell proliferation was tested at 72 h by measuring [3H]-thymidine incorporation on DNA (see Supporting Materials and Methods).

### EXAMPLE 1. Structure-based pharmacophore model for identifying new Calcineurin-NFATc signaling inhibitors

In order to identify compounds that inhibit CN-NFATc signaling, without interfering with general CN phosphatase activity, the crystal structure of the AKAP79-derived peptide (EPIAIIITDTE; SEQ ID NO: 3) in complex with CN was used to develop a structure-based pharmacophore model. In this complex, the side chains of Ile8, Ile9 and Ile10 of the peptide form hydrophobic interactions with CNA residues Tyr288 and Ile331, with Val328, and with Met290, Phe299, Pro300 and Met329, respectively; while the side chain of Thr11 forms a hydrogen bond interaction (yellow dashes) with Asn330 of CNA. Thus, with the aim of reproducing these key interactions, the pharmacophore model includes five hydrophobic features positioned over Ile8 (C_{δ1} and C_{γ2} atoms), Ile9 (C_{δ1} atom) and Ile10 (C_{δ1} and C_{γ2} atoms), and a hydrogen bond acceptor feature positioned over the O_{γ1} atom of Thr11. In addition, the β strand conformation of the peptide enlarges the of β sheet of CAN by forming the characteristic backbone-backbone hydrogen bond interactions. Accordingly, three hydrogen bond acceptor features (green spheres and arrows) are positioned over the oxygen atoms of the backbone carbonyl groups of Ala7, Ile9 and Thr11, and two hydrogen bond donor features (magenta arrows and spheres) are positioned over the nitrogen atoms of the backbone N-H groups of Ile9 and Thr11 to reproduce the interactions with the β14-strand of CNA. Finally, the presence of the polar Gln333 in CNA, near the hydrophobic groove, led us to add an extra hydrogen bond donor feature (magenta arrow and sphere) at 2.8 Å from the O atom of Gln333. In contrast to large peptides, small non-peptidic molecules cannot fulfill these twelve pharmacophoric features. Thus, 864 different pharmacophore models, containing at least seven of these features, were created.

### EXAMPLE 2. A pharmacophore-based victual screening of small, non-peptidic ligands that bind to the PxIxIT binding pocket of Calcineurin A

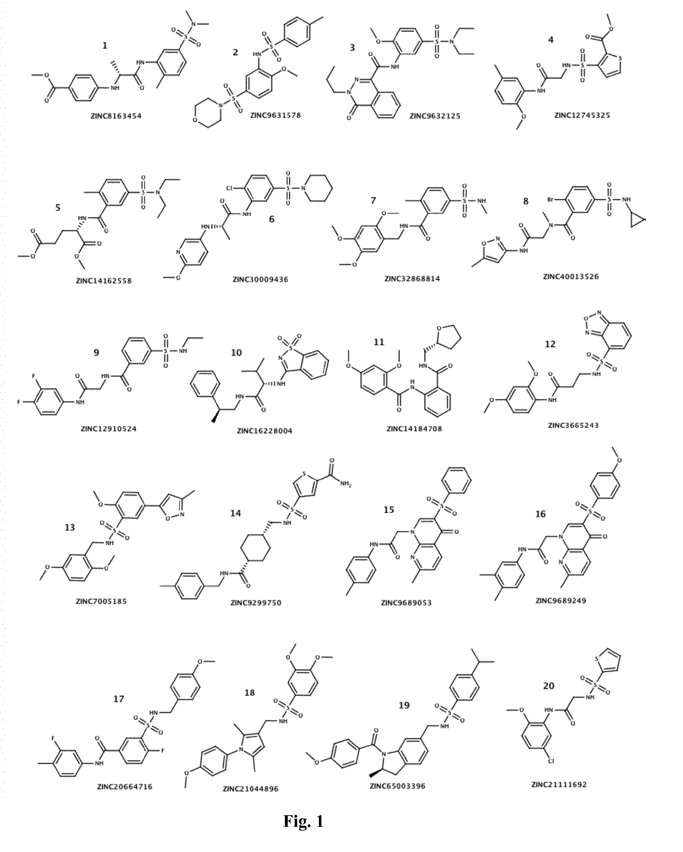

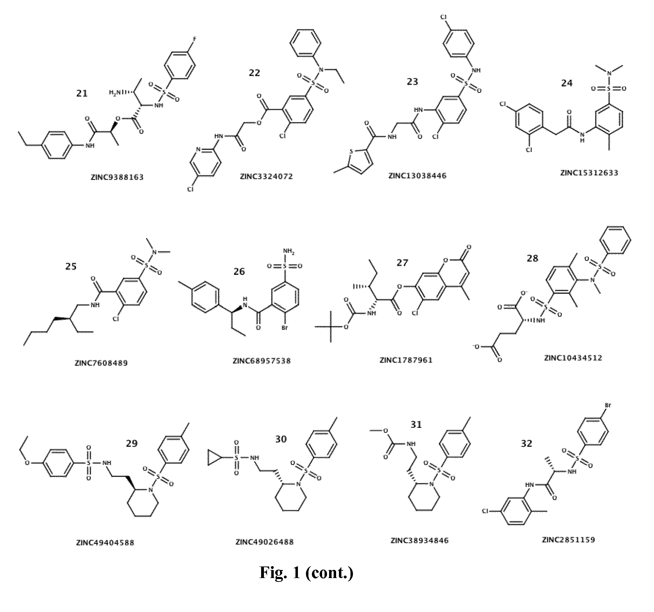

A ligand dataset of about 5.5 million commercially available "lead-like" compounds was compiled from the ZINC database (J. J. Irwin, and Shoichet, J. Chem. Inf. Model., 2005, 45, 177-182) (see Materials and Methods). Diverse conformations (between 150 and 250 per molecule, depending on size) of these about 5.5 million molecules were rigid-body fitted to the 864 pharmacophore models, resulting in a total of about 160 thousand putative inhibitors. These were ranked from best to worst fitting and about 1.7 thousand compounds with fit values better than 3.0 were mapped again onto the pharmacophore models using a flexible fitting method, which slightly modifies each conformation for enhanced fitting. Finally, 641 compounds with flexible fit values higher than 4.5 were inspected visually for chemical complementation between the ligand and CNA, for toxicity, and for similarity. These procedures yielded thirty-two diverse molecules for experimental evaluation (Fig. 1).

### EXAMPLE 3. Displacement of NFATc from the Calcineurin-NFATc interaction,

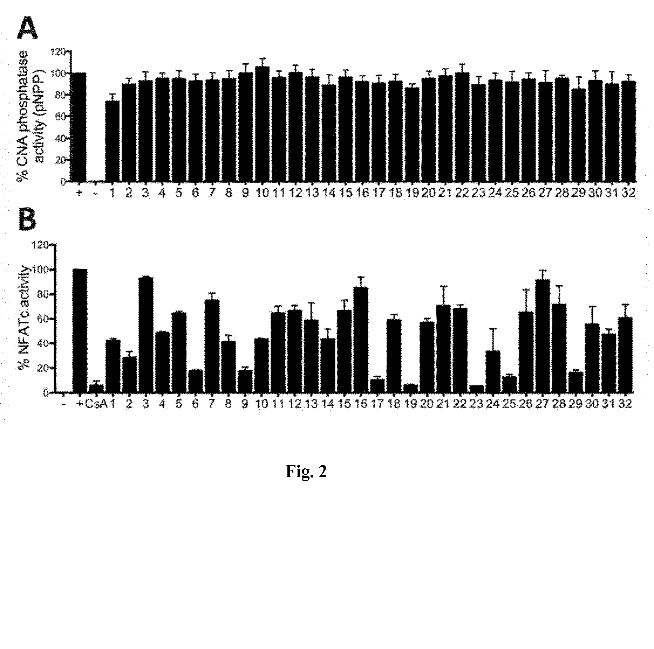

Potential immunosuppressant CN inhibitors should compete with NFATc for binding, without blocking the general phosphatase activity of CN. As an initial screening test, the phosphatase activity of CN was analyzed using the small molecule p-nitrophenil phosphate (pNPP) as substrate (see Material and Methods). With the exception of 1, none of the other compounds affect CN phosphatase activity towards pNPP (Fig. 2A). In contrast, ZnCl₂, which was used as a negative control, strongly inhibits enzymatic activity (Fig. 2A). The ability of compounds 1-32 in inhibiting CN-NFATc signaling was evaluated in human HEK 293T cells using a luciferase gene reporter assay that includes the NFATc-dependent IL2 promoter (3xNFATc-luc). After 24 hours of treatment, 50 µM of compounds 6, 9, 17, 19, 23, 25 and 29 inhibit more than 75% NFATc activity, while compounds 1, 2, 4, 8, 10, 14, and 24 inhibit 50-75% NFATc activity (Fig. 2B).

In order to ascertain that the inhibition of the NFATc activity achieved by compounds 6, 9, 17, 19, 23, 25 and 29 was due to specific competition with the PxIxIT motif of NFATc for binding to CN, additional experimental approaches were carried out. First, none of these compounds compete against the phosphoRII peptide, a CN substrate derived from the protein substrate type IIa regulatory subunit of protein kinase A, that binds the LxVP binding site of CN (Fig. 3A). Thus, consistent with previous results (Fig. 2A), these compounds do not bind the LxVP binding site of CN. Second, we analyzed whether increasing concentrations of these molecules were able to compete with NFATc2 (GST-NFATc2) for binding to FLAG-CNA (Fig. 3B). Clearly, all compounds disrupt CNA-NFATc2 interaction, with compounds 17, 19, 23, and 25 achieving the highest displacement. Inactive compound 3 was used as a negative control and the RCAN3-derived CIC peptide (Mulero et al., 2009, *sura*.), which includes a PxIxIT sequence, as a positive control. And third, competitive binding studies between the NFATc2-derived SPRIEIT peptide and compounds 6, 9, 17, 19, 23, 25 and 29 for binding to CNA were performed by fluorescence anisotropy assays. Compounds 17, 19, 23, and 25 are the most potent competitors with IC50 values within the 7-specifically displace NFATc2 binding to CN, without blocking the general phosphatase activity of CN.

### EXAMPLE 4: Compounds 17, 19, 23, and 25 inhibit NFATc2 activation and translocation to the nucleus.

Activation of CN leads to dephosphorylation of NFATc transcription factors and their translocation from the cytosol into the nucleus. Compounds 6, 9, 17, 19, 23, 25 and 29 inhibited NFATc2 dephosphorylation at 100 µM, while this effect is maintained at 10 µM for 19, 23 and 25 (Fig. 4A). Next, we examined whether the modulatory effect on NFATc2 dephosphorylation was accompanied by inhibition of its translocation into the nucleus. Activation of CN in U2OS cells with ionomycin/ CaCl2 (Io + Ca2+), when transfected with HA-NFATc2-EGFP fusion protein, causes rapid translocation of NFATc2 into the nucleus (Fig. 4B). Compounds 6, 9, 17, 19, 23, 25, 29 inhibited this translocation of NFATc2 at 100 µM. However, 19, 23 and 25 inhibit EGFP-NFATC translocation at 10 µM to a greater extent than 6, 9, 17, and 29 (Fig. 4B).

Dose-response assays of NFATc-dependent luciferase reporter gene were carried out, with the aim of evaluating the ability of these compounds to induce NFATc binding to promoter regions of target genes that lead to gene expression. HEK 293T cells co-transfected with plasmids 3xNFATc-luc and pRL-null as an internal control of transfection, were pretreated with increasing concentrations of 6, 9, 17, 19, 23, 25, 29 and stimulated with Io/PMA/Ca2+ prior to luciferase activity measurement. All compounds inhibited NFATc activity in a dose-dependent manner for concentrations ranging from 100 µM to 1,5 µM (Fig. 4C). Almost no cytotoxicity effects were associated at these concentrations (Fig. 6), which indicates that the inhibition of NFATc activity is due to the presence of 6, 9, 17, 19, 23, 25 and 29.

### EXAMPLE 5: Compounds 17, 19, 23, and 25 inhibit Calcineurin-NFATc signaling and proliferation in activated human primary CD4+ T-cells

Translocation of dephosphorylated NFATc into the nucleus triggers, in cooperation with other inducible transcription factors, the expression of NFATc-dependent genes such as interleukin 2 (IL2), gamma interferon (IFNγ) and alpha tumor necrosis factor (TNFα) among others. In this context, we measured cytokine mRNA levels and protein production in human CD4+ from peripheral blood lymphocytes (hPBL) stimulated with anti-CD3/anti-CD28 beads. Compounds 17, 19, 23 and 25 were able to interfere with downstream signaling of CN-NFATc pathway (Fig. 5). We observed that IL2, IFNγ and TNFα mRNA levels were significantly decreased in the presence of all four compounds (Fig. 5A). In addition, the release of IL2 and IFNγ by human CD4+ cells to the cell supernatant was also dramatically reduced (Fig. 5B). Furthermore, T cell proliferation was also inhibited in a dose dependent manner (Fig. 5C). In conclusion, compounds 17, 19, 23 and 25 inhibit NFATc-dependent cytokine production, secretion and proliferation of CD4+ hPBLs cells, therefore having immunosuppressant activity.

## Claims

1. A compound of formula (I) 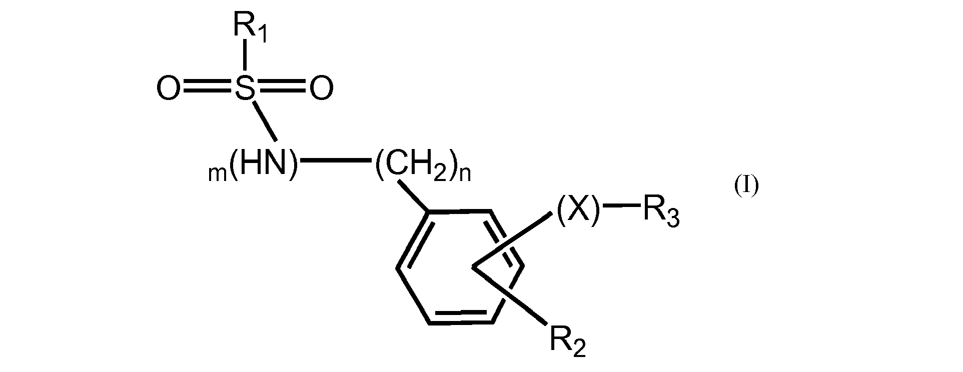 wherein:
R₁ is
- hydrogen, halogen, hydroxy, trihalomethyl, trihalomethoxy, carbamoyl, phenyl or cyano,
- (C₁-C₈) alkyl,
- (C₁-C₈) alkylamino,
- (C₁-C₈) dialkylamino,
- (C₂-C₈) alkenyl,
- (C₂-C₈) alkenylamino,
- (C₂-C₈) dialkenylamino,
- (C₂-C₈) alkinyl,
- (C₂-C₈) alkinylamino,
- (C₂-C₈) dialkinylamino,
- alkoxy,
- aralkyl,
- alkanoyl,
- a phenyl group or a phenylamino group, wherein the phenyl group is optionally substituted with 1, 2, 3, 4 or 5 substituents Y, which can be the same or different, and wherein Y is chosen from alkyl, alkoxy, halogen, hydroxy, trihalomethyl, trihalomethoxy, carbamoyl, phenyl and cyano,
- a benzyl group or a benzylamine group, wherein the phenyl ring of the benzyl group is optionally substituted with 1, 2, 3, 4 or 5 substituents Y as defined above which can be the same or different,
- a 5-membered carbocyclic or heterocyclic group optionally substituted with 1, 2, 3 or 4 substituents Y as defined above which can be the same or different,
- a 6-membered carbocyclic or heterocyclic group optionally substituted with 1, 2, 3, 4 or 5 substituents Y as defined above which can be the same or different,
- a group having the structure 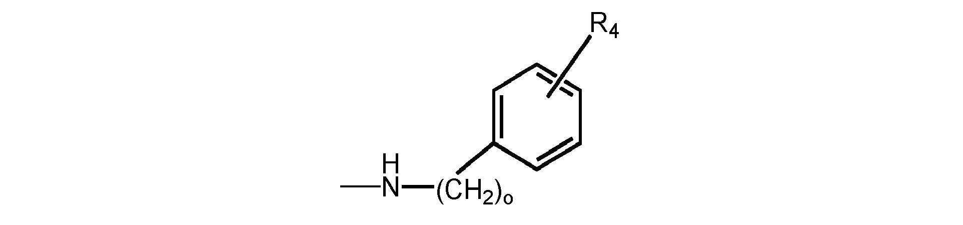 wherein o is an integer from 0 to 10, and R₄ is alkyl, alkoxy or halogen; or
- a group having the structure 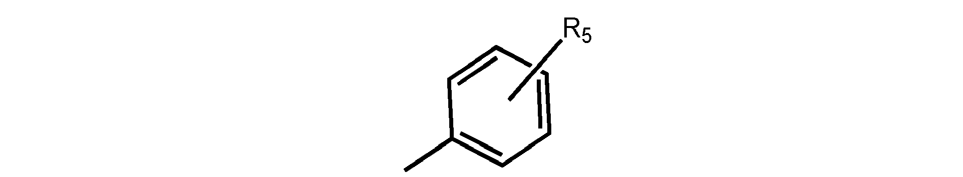 wherein R₅ is an alkyl group,
m is 0 or 1,
n is 0, 1 or 2,
X is -NH-CO- or -CO-NH-,
R₂ is hydrogen, halogen, alkyl, alkoxy, hydroxy, trihalomethyl, trihalomethoxy, carbamoyl, phenyl or cyano,
R₃ is
- alkyl,
- alkoxy or
- a group having the structure -A-B
wherein
A is -(CH(R₆))-NH-, -CO-, -CH₂-CO-NH-, -CH(R₆)-NH-CO- or a direct bond, wherein R₆ is hydrogen or methyl, and
B is an aromatic 5-membered carbocyclic or heterocyclic group optionally substituted with 1, 2, 3 or 4 substituents Y as defined above which can be the same or different or an aromatic 6-membered carbocyclic or heterocyclic group optionally substituted with 1, 2, 3, 4 or 5 substituents Y as defined above which can be the same or different, or wherein R₂ and R₃ form a heterocyclic group which is condensed to the phenyl group in formula (I) to form a bicyclic structure and wherein the heteroatom in the heterocycle is the nitrogen atom in the X group, said heteroatom being substituted with a group having the structure -A-B, wherein A and B are as defined above, or a compound having the structure 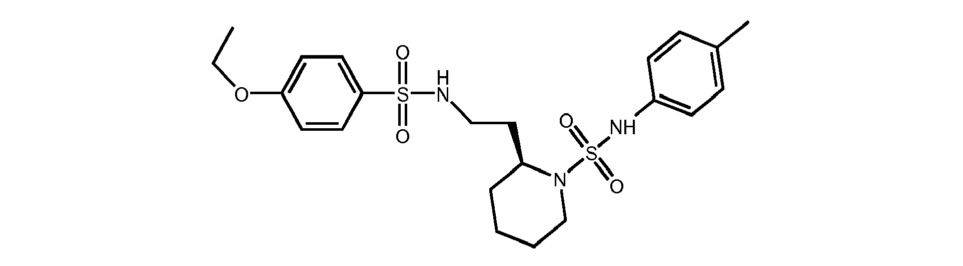 or a pharmaceutically acceptable salt, hydrate, ester, solvate, prodrug, metabolite, stereoiosomer or mixtures thereof for use in the treatment of a disease in a subject, wherein said disease is selected from the group consisting of an inflammatory disease, an autoimmune disorder, a cardiovascular disease, a neurodegenerative disease, a disease occurring with uncontrolled cell proliferation and/or differentiation, an angiogenesis-related disease, an allergy, anaphylaxis and alopecia.

2. The compound for use according to claim 1, wherein R₁ is selected from the group consisting of:
- a N-piperidine group,
- an alkylamino group,
- a dialkylamino group,
- a group having the structure 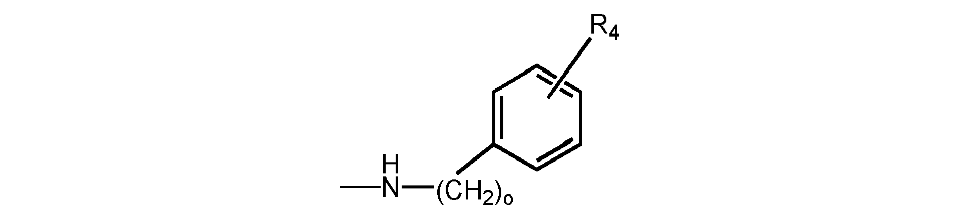 wherein if o is 1 then R₄ is a methoxy group or wherein if o is 0 then R₄ is chlorine, and
- a group having the structure 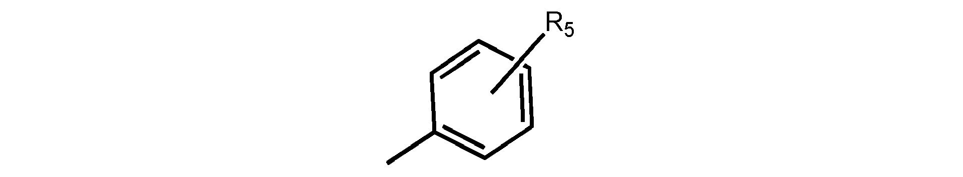 wherein R₅ is an alkyl group.

3. The compound for use according to claim 2, wherein the R₁ is a group having the structure

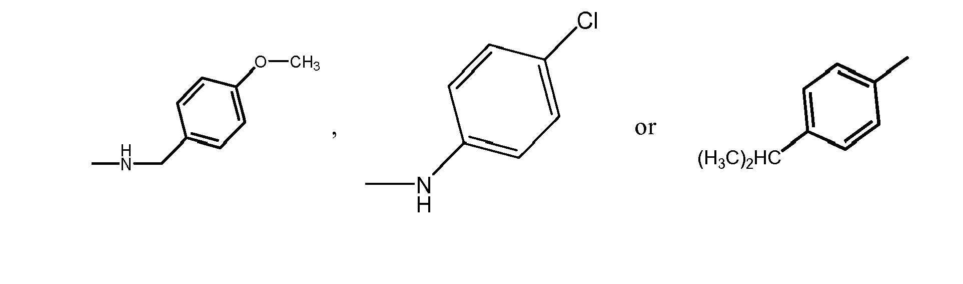

4. The compound for use according to any of claims 1 to 3, wherein m and n are both 0 or 1 or wherein m is 1 and n is 2.

5. The compound for use according to any of claims 1 to 4, wherein R₂ is chlorine, fluorine or hydrogen.

6. The compound for use according to any of claims 1 to 5, wherein R₃ is a group having the structure -A-B wherein A is -(CH(R₆))-NH- and R₆ is as defined above, and B is a 6-membered heterocyclic group optionally substituted with 1, 2, 3, 4 or 5 substituents Y as defined above which can be the same or different.

7. The compound for use according to any of claims 1 to 5, wherein R₃ is a group having the structure -A-B wherein A is -CH₂-CO-NH- or a direct bond and B is a phenyl optionally substituted with 1, 2, 3, 4 or 5 substituents Y as defined above which can be the same or different.

8. The compound for use according to any of claims 1 to 5, wherein R₃ is a group having the structure -A-B wherein A is -CH(R₆)-NH-CO- and B is a 5-membered heteroaryl group optionally substituted with 1, 2, 3 or 4 substituents Y as defined above which can be the same or different.

9. The compound for use according to any of claims 6 to 8, wherein the -A-B group has the structure 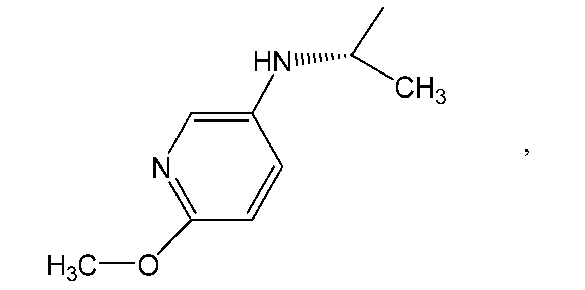 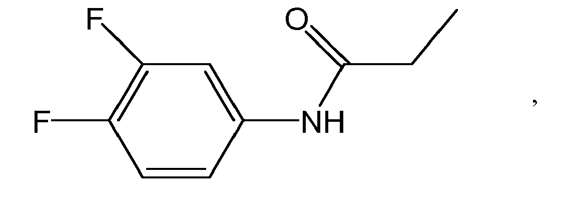 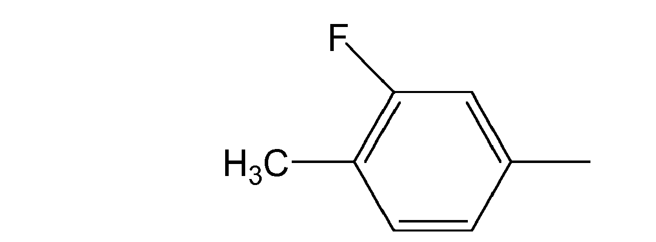 Or

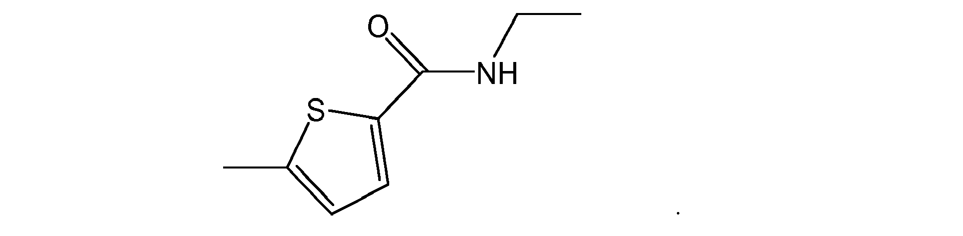

10. The compound for use according to any of claims 1 to 5, wherein the R₃ group is a C₈ alkyl group having the structure

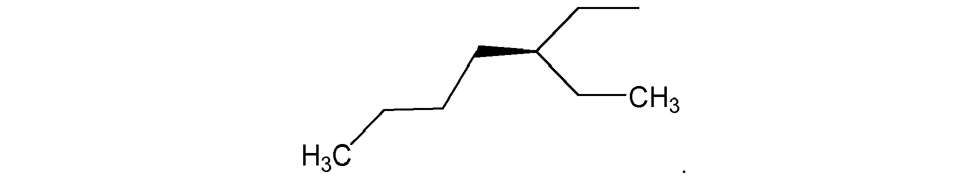

11. The compound according to claim 1, wherein R₂ and R₃ form a heterocyclic group which is condensed to the phenyl group in formula (I) to form a bicyclic structure wherein the nitrogen atom in the X group is the heteroatom in the heterocycle, said heteroatom being substituted with a group having the structure -A-B, wherein A and B are as defined above.

12. The compound according to claim 11, wherein the bicyclic structure has the following structure

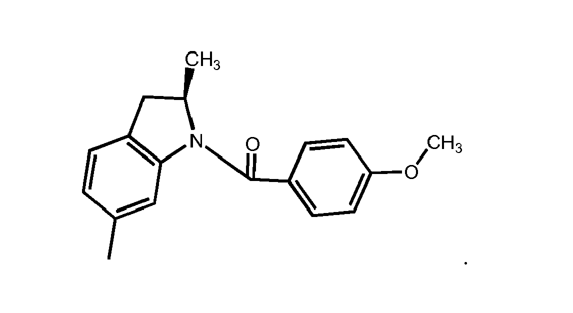

13. The compound for use according to any of claims 1 to 12, wherein the compound is selected from the group consisting of: 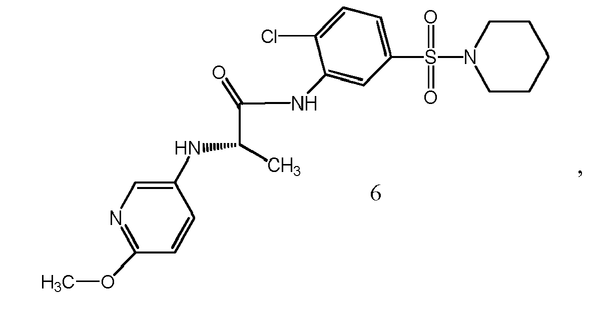 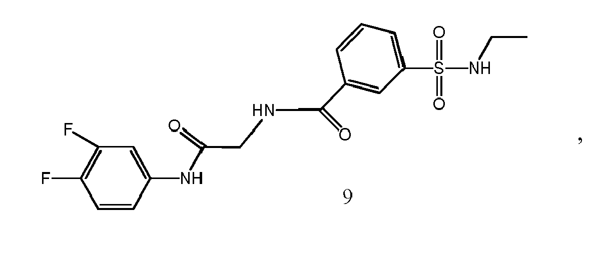 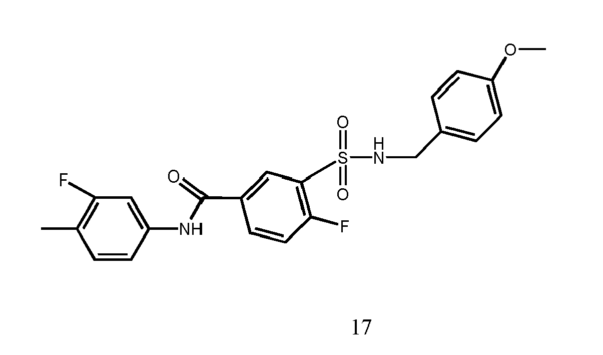 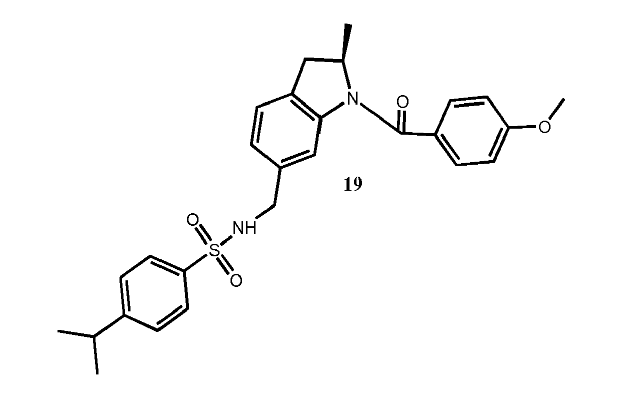 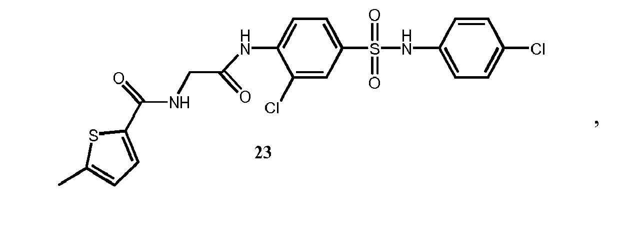 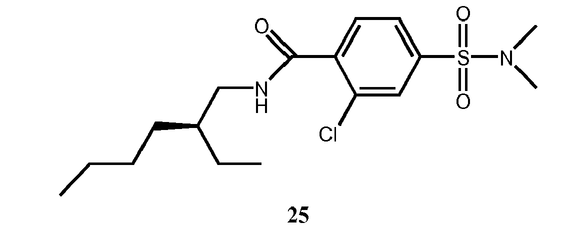 and

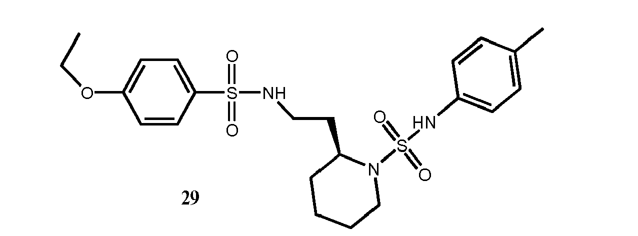

14. The compound for use according to any of claims 1 to 13, wherein the cardiovascular disease is cardiac hypertrophy or atherosclerotic plaque instability, wherein the disease occurring with uncontrolled cell proliferation and/or differentiation is malignancy and tumor progression, wherein the angiogenesis related disease is cancer, abnormal angiogenesis in diabetic subjects, myocardial edema or pulmonary edema and/or wherein the allergic disease is anaphylaxis.


SEQUENCE LISTING

<110>  FUNDACIÓ INSTITUT DE RECERCA BIOMÈDICA DE BELLVITGE
       UNIVERSITAT AUTÒNOMA DE BARCELONA

<120>  THERAPEUTIC USES OF NON-PEPTIDE INHIBITORS OF THE CALCINEURIN -
       NFAT SIGNALLING PATHWAY

<130>  P12223EP00

<160>  3

<170>  PatentIn version 3.5

<210>  1
<211>  16
<212>  PRT
<213>  Artificial Sequence

<220>
<223>  NFATc2 derived peptide

<400>  1

Ala Ser Gly Leu Ser Pro Arg Ile Glu Ile Thr Pro Ser His Glu Leu
1               5                   10                  15


<210>  2
<211>  21
<212>  PRT
<213>  Artificial Sequence

<220>
<223>  RCAN3178-203 (CIC) peptide

<400>  2

Lys Tyr Glu Leu His Ala Gly Thr Glu Ser Thr Pro Ser Val Val Val
1               5                   10                  15


His Val Cys Glu Ser
            20


<210>  3
<211>  11
<212>  PRT
<213>  Artificial Sequence

<220>
<223>  AKAP79-derived peptide

<400>  3

Glu Pro Ile Ala Ile Ile Ile Thr Asp Thr Glu
1               5                   10